(19) Europäisches Patentamt
European Patent Office
Office européen des brevets

(11) Publication number: **0 334 477 A2**

# (12) EUROPEAN PATENT APPLICATION

(21) Application number: 89301430.8

(22) Date of filing: 15.02.89

(51) Int. Cl.[4]: C07D 307/02 , C07D 207/02 , G03C 1/733 , C07D 307/78 , C07D 409/06 , C07D 409/14 , C07D 405/06

(30) Priority: 19.02.88 GB 8803881

(43) Date of publication of application: 27.09.89 Bulletin 89/39

(84) Designated Contracting States: ES GR

(71) Applicant: TRAQSON LIMITED
45 London Wall
London EC2M 5TE(GB)

(72) Inventor: Heller, Harry George
135 Pencisely Road
Cardiff Wales(GB)
Inventor: Whittall, John
19 Pen-y-Alt Watford Park
Caerphilly Wales(GB)

(74) Representative: Ritter, Stephen David et al
Mathys & Squire 10 Fleet Street
London EC4Y 1AY(GB)

## (54) Novel photoactive compounds, processes for their production and intermediates therefor.

(57) Photoactive compounds having the general formulae below are provided

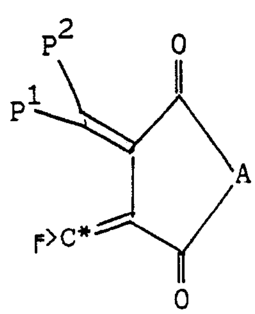

or

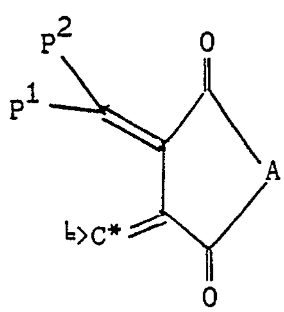

(IIIa) (IIIb)

in which A represents oxygen or specified imido groups;
$P^1$ represents a 3-furyl, a 3-thienyl, a 3-pyrryl, a 3-benzofuryl or a 3-benzothienyl group, said 3-furyl, 3-thienyl and 3-pyrryl groups which is unsubstituted or substituted in the 2-position and/or the 5-position, with specified substituents;
$P^2$ represents alkyl having 1 to 20 carbon atoms, cycloalkyl having 3 to 12 carbon atoms, aralkyl having 7 to 9 carbon atoms, aryl having 6 to 14 carbon atoms which may be unsubstituted or substituted with one or more halogen atoms, or alkaryl having 7 to 22 carbon atoms; and
$_{F}>C^*$ and $^{E}>C^*$ represent a substituted or unsubstituted bridged polycyclic hydrocarbon residue containing from 7 to 20 carbon atoms in a polycyclic system, said residue having a plane of asymmetry which is parallel to the plane which includes the single bonds extending from carbon atom $C^*$ and the

EP 0 334 477 A2

anhydride or imide ring, any substituents on the bridged polycyclic hydrocarbyl residue being selected from alkyl groups having 1 to 4 carbon atoms, halogen atoms and hydroxy groups.

# NOVEL PHOTOACTIVE COMPOUNDS, PROCESSES FOR THEIR PRODUCTION AND INTERMEDIATES THEREFOR

The invention relates to a novel class of photoactive compounds and to processes for their production. The invention also includes novel intermediates useful in the production of the photoactive compounds and a novel condensation procedure of general applicability.

As used herein, the expression "photoactive compound" is defined as a compound which undergoes a structural change (hereinafter referred to as a "photoactive change") when subjected to electromagnetic radiation of a selected wavelength or wavelengths and undergoes a reversal to its original structure when subjected to thermal energy or to electromagnetic radiation of another wavelength and wherein the distinct structural forms interact differently with electromagnetic radiation. For example they may have different spectral absorption characteristics which can manifest themselves by the structural forms having different observable colours and often different dispersivities and refractive indices.

Photoactive compounds as defined above which undergo major colour changes on conversion between their respective structural forms are referred to as "photochromic compounds".

Photochromic compounds have a wide range of uses, e.g. in the manufacture of image and data recording media and in display systems.

An important class of photochromic compounds is based on bismethylenesuccinic anhydride or "fulgide" (I).

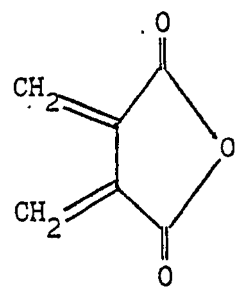

(I)

Such compounds are exemplified by those described in GB-1442628, GB 1464603, GB 2002752 and US 4220708. Thus, for example US-4220708 describes and claims a class of compounds of the formula

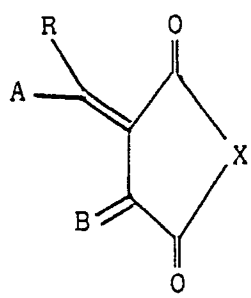

(II)

wherein X represents oxygen or $NR_6$, $R_6$ being hydrogen or an alkyl, aryl or aralkyl group;
R represents an alkyl or aryl group;
A represents a 3-furyl, 3-thienyl, 3-benzofuryl or 3-benzothienyl group; and
B represents an adamantylidene group or the grouping

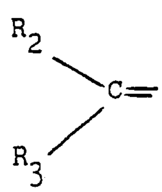

in which $R_2$ and $R_3$ independently represent an alkyl, aryl, or a heterocyclic group containing a 3-furyl or 3-thienyl ring or one of $R_2$ and $R_3$ represents hydrogen and the other represents an alkyl or aryl group.

By appropriate selection of substituents on the 3-furyl, 3-thienyl, 3-benzofuryl and 3-benzothienyl groups represented by A and by appropriate selection of groups X, R and B, photochromic compounds having a range of properties may be produced. Compounds of formula II in which B represents an adamantylidene group are preferred as they have a high quantum efficiency for bleaching of their coloured forms. They further show good thermal stability and the ability to undergo a large number of colour change cycles without substantial deterioration in the character of the absorption spectra as a result of the formation of so-called "fatigue products" or irreversible side reactions.

However the preferred compounds of US 4220708 in which B represents adamantylidene are expensive to produce owing to the high cost of adamantanone used as a starting material. Also, for the known compounds and their coloured forms, only a relatively restricted number of characteristic wavelengths may be used to induce a photochromic change in structure and compounds having only a relatively restricted range of characteristic absorption wavelengths are available. These disadvantages impose limitations, where for example it is wished to mass-produce data storage media economically, or where it is necessary to produce a photochromic compound having optical characteristics which match the characteristic wavelengths of available sources of electromagnetic radiation, e.g. semi-conductor diode lasers.

A further difficulty with many known compounds is their limited solubility in organic solvents and limited capacity to form solid solutions with plastics materials. Photochromic compounds having a higher solubility than known compounds would be desirable in many fields. For example in the production of security printing inks and varnishes it is desirable for the components of the inks to be soluble in the liquid ink base. In other methods of printing, higher solubility would enhance the formulation of solid solutions in plastics materials. Similarly in order to produce thin, uniform coatings on data storage media, it is advantageous to use a coating fluid containing a photochromic compound in the dissolved state. Currently available photochromic materials of limited solubility fail to satisfy these criteria.

We have now developed a novel class of photochromic materials which overcome or reduce such disadvantages of the known materials.

According to one aspect of the present invention there are provided photochromic compounds having the general formula

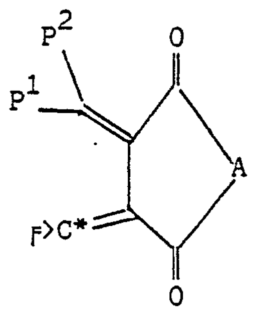

or

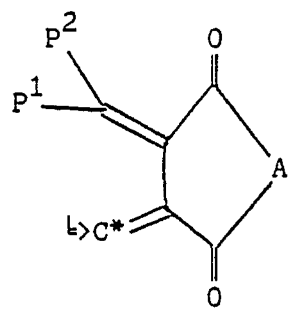

(IIIa) (IIIb)

wherein the substituents $P^1$, $P^2$, F>C*, L>C* and A are defined as follows:

A represents oxygen or $NR^1$ wherein $R^1$ represents hydrogen, alkyl having 1 to 20 carbon atoms, cycloalkyl having 5 to 12 carbon atoms, aralkyl having 7 to 9 carbon atoms, aryl having 6 to 14 carbon atoms, which may be substituted with one or more halogen or alkoxy groups having 1 to 20 carbon atoms, or alkaryl having 7 to 22 carbon atoms;

$P^1$ represents a 3-furyl, a 3-thienyl, a 3-pyrryl, a 3-benzofuryl or a 3-benzothienyl group, each of which may be unsubstituted or substituted by substituents defined below;

$P^2$ represents a $C_{1-20}$ alkyl, a $C_{3-12}$ cycloalkyl, a $C_{7-9}$ aralkyl, a $C_{6-14}$ aryl (which may be unsubstituted or substituted by one or more halogen atoms) or a $C_{7-22}$ alkaryl group; and

F>C* and L>C* represent a substituted or unsubstituted bridged polycyclic hydrocarbon residue containing from 7 to 20 carbon atoms in a polycyclic system, said residue having a plane of asymmetry which is parallel to the plane which includes the single bonds extending from carbon atom C* and the anhydride or imide ring, any substituents on the bridged polycyclic hydrocarbyl residue being selected from

alkyl groups having 1 to 4 carbon atoms, halogen atoms and hydroxy groups.

Definitions of Optional Substituents

The optional substituents on the 3-furyl, 3-thienyl and 3-pyrryl groups may be in the 2- and/or 5-positions and may be the same or different and the optional substituents on the 3-benzofuryl and 3-benzothienyl groups may be in the 2-position.

These substituents are defined as follows:

The optional 2-substituents are selected from $C_{1-20}$ alkyl groups and $C_{7-12}$ aralkyl groups;

The optional 5-substituents are selected from

(i) $C_{1-20}$ alkyl groups,

(ii) $C_{3-12}$ cycloalkyl groups,

(iii) $C_{3-12}$ cycloalkenyl groups,

(iv) $C_{6-14}$ aryl groups, which are unsubstituted or substituted

(a) with one or more alkoxy groups,

(b) with a group of the formula $-NR_7R_8$, wherein $R_7$ and $R_8$ each represent hydrogen or $C_{1-20}$ alkyl or together with the nitrogen to which they are attached represent a 1-pyrrolidine, a 1-piperidine or a 1-morpholine group), or

(c) with one or more halogen atoms,

(v) $C_{7-12}$ aralkyl groups,

(vi) halogen atoms, and

(vii) heterocyclic groups selected from 2-thienyl, 3-thienyl, 2-furyl and 3-furyl groups, said heterocyclic groups (vii) being unsubstituted or substituted by one or more $C_{1-3}$ alkyl groups or halogen atoms.

Preferred unsubstituted or substituted $C_{6-14}$ aryl groups within definition (iv) are unsubstitued or substituted phenyl groups. In such substituted phenyl groups, the alkoxy substituents (a) and the halogen substituents (c) may be in the ortho and/or para positions and the $-NR_7R_8$ substituent (b) may be in the para position. In full, the substituents $P^1$, $P^2$, ╒>C*, ╘>C* and A in the compounds of formulae (IIIa) and (IIIb) are defined, in accordance with the invention, as follows:

A represents oxygen or $NR^1$ wherein $R^1$ represents hydrogen, alkyl having 1 to 20 carbon atoms, cycloalkyl having 5 to 12 carbon atoms, aralkyl having 7 to 9 carbon atoms, aryl having 6 to 14 carbon atoms, which may be substituted with one or more halogen or alkoxy groups having 1 to 20 carbon atoms, or alkaryl having 7 to 22 carbon atoms;

$P_1$ represents a 3-furyl, a 3-thienyl, a 3-pyrryl, a 3-benzofuryl or a 3-benzothienyl group, said 3-furyl, 3-thienyl and 3-pyrryl groups being unsubstituted or substituted in the 2-position and/or the 5-position, and said 3-benzofuryl and 3-benzothienyl groups being unsubstituted or substituted in the 2-position, said optional 2-substituents being selected from alkyl groups having 1 to 20 carbon atoms and aralkyl groups having 7 to 12 carbon atoms, and said optional 5-substituents being selected from (i) alkyl groups having 1 to 20 carbon atoms, (ii) cycloalkyl groups having 3 to 12 carbon atoms, (iii) cycloalkenyl groups having 3 to 12 carbon atoms, (iv) aryl groups having 6 to 14 carbon atoms, which aryl groups may be unsubstituted or substituted with (a) one or more alkoxy groups, (b) with a group of the formula $-NR_7R_8$ wherein $R_7$ and $R_8$ each represent hydrogen or alkyl having 1 to 20 carbon atoms or together with the nitrogen atom to which they are attached represent a 1-pyrrolidine, a 1-piperidine, or a morpholine group, or (c) with one or more halogen atoms, (v) aralkyl groups having 7 to 12 carbon atoms, (vi) halogen atoms and (vii) heterocyclic groups selected from 2-thienyl, 3-thienyl, 2-furyl and 3-furyl groups, said last-mentioned 2-thienyl, 3-thienyl, 2-furyl and 3-furyl groups being unsubstituted or substituted by one or more $C_{1-3}$ alkyl groups or halogen atoms;

$P^2$ represents alkyl having 1 to 20 carbon atoms, cycloalkyl having 3 to 12 carbon atoms, aralkyl having 7 to 9 carbon atoms, aryl having 6 to 14 carbon atoms which may be unsubstituted or substituted with one or more halogen atoms, or alkaryl having 7 to 22 carbon atoms; and

╒>C* and ╘>C* represent a substituted or unsubstituted bridged polycyclic hydrocarbon residue containing from 7 to 20 carbon atoms in a polycyclic system, said residue having a plane of asymmetry which is parallel to the plane which includes the single bonds extending from carbon atom C* and the anhydride or imide ring, any substituents on the bridged polycyclic hydrocarbyl residue being selected from

alkyl groups having 1 to 4 carbon atoms, halogen atoms and hydroxy groups.

In a preferred class of compounds according to the invention the symbols ⌈>C* and ⌊>C* represent the structure

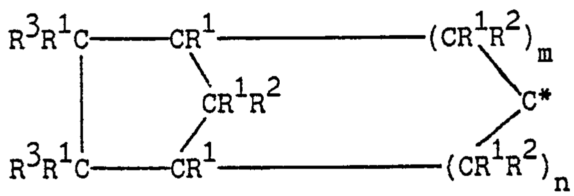

(IV)

wherein each $R^1$ and $R^2$ is independently selected from hydrogen and $C_{1-4}$ alkyl, each $R^3$ is independently selected from hydrogen and $C_{1-4}$ alkyl or the two $R^3$ groups together form an alkylene group having up to 6 carbon atoms, and one of m and n is 1 and the other is 1 or 0. Most preferably, one of m and n is 1 and the other is 0. In a particularly preferred class of compounds according to the invention, the symbols ⌈>C* and ⌊>C* represent the structure

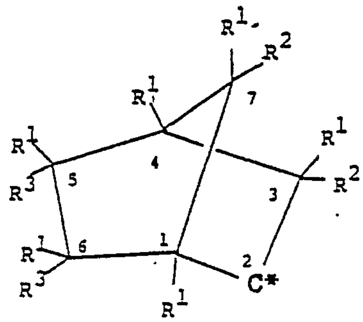

(IV')

where each of the $R^1$, $R^2$ and $R^3$ substituents is as defined above. In the above formula the $R^1$, $R^2$ and $R^3$ substituents are each preferably hydrogen or methyl. Most preferably one, two or three of the carbon atoms at positions 1, 4, 5 and 6 is substituted by a methyl group and the remaining substituents $R^1$, $R^2$ and $R^3$ are all hydrogen.

Specific examples of polycyclic hydrocarbyl residues represented by ⌈>C* and ⌊>C* includes the following

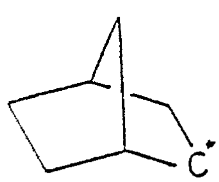

Va

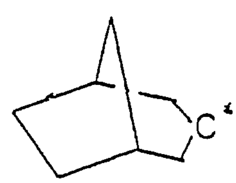

Vb

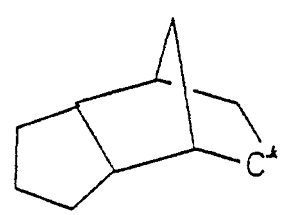

Vc

and

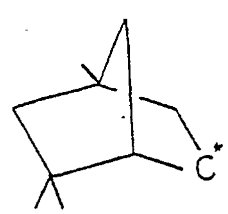

Vd

It will be appreciated that compounds of formula (III) can exist in a plurality of isomeric forms. Thus, for example, the two isomers

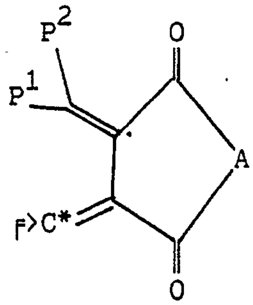

(IIIa)

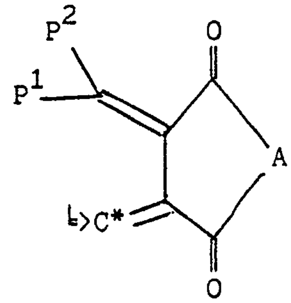

(IIIb)

are geometric isomers in which the groups F>C* and L>C* represent the two possible modes of attachment of the asymmetric polycyclic hydrocarbyl residue to the ring structure containing moiety A. These isomers are not readily interconvertible because the double bonds linking the groups F>C* and L>C* to the remainder of the molecule prevent rotation of the groups F>C* and L>C*. They may, however, be interconverted by irradiating with ultraviolet light.

In addition to possible geometric isomerism, two additional forms of isomerism can occur.

One such form of isomerism occurs as a result of the groups F>C* and L>C* existing in enantiomorphic (mirror image) forms.

For example in the case of structure IIIa the enantiomorphic forms F>C* and ꟻ>C* of the asymmetric polycyclic hydrocarbyl residue enable the following structures to be written:

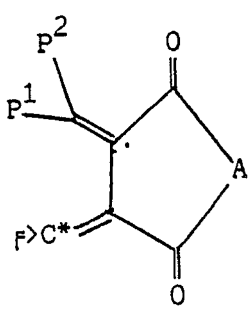

(IIIa)

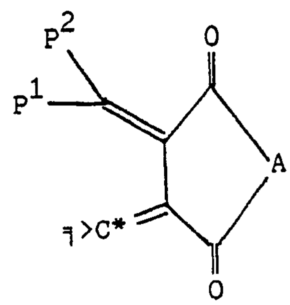

(IIIa')

Similarly in the case of structure IIIb the enantiomorphic forms ⅃>C* and L>C* of the asymmetric polycyclic hydrocarbyl residue enable the following structures to be written

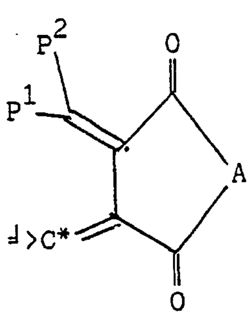

(IIIb)

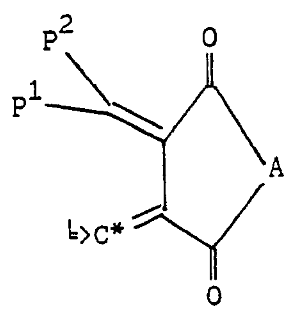

(IIIb')

The different isomeric forms can exhibit diverse photochromic properties in view of different interactions between the asymmetric polycyclic hydrocarbyl residue and the group $P^1$.

Thus, for example where ⌐>C* and L>C* represent structure Va in each of its two possible modes of attachment, the following geometric isomers can be drawn:

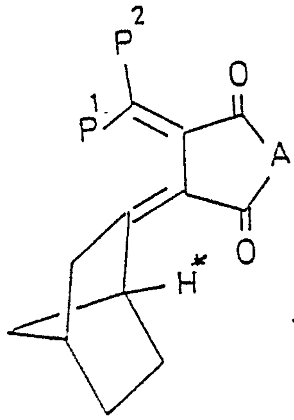

VIa

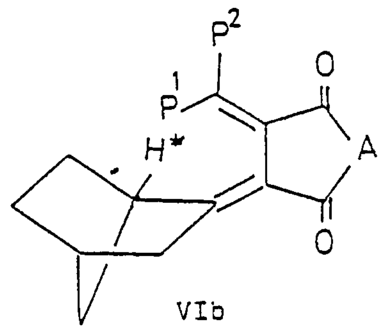

VIb

it will be apparent that in one of these isomers, the methine hydrogen H* is relatively close to $P^1$, whereas in the other, the methine hydrogen H* is relatively close to a carbonyl of the five-membered ring.

A further form of isomerism results from the capacity of the compounds of formulae IIIa and IIIb to exist in different helical conformational forms, either in the compounds of formulae IIIa and IIIb themselves, which give rise to different cyclised (differently coloured) spiro structural forms, resulting from the helical conformers being exposed to electromagnetic radiation of the required selected wavelength, or in the spiro structural forms formed on cyclisation.

The helical forms of the compound (IIIa) may be represented by the following structures:

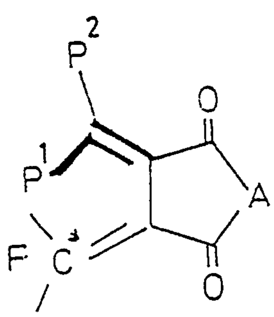

(IIIa<u>a</u>)

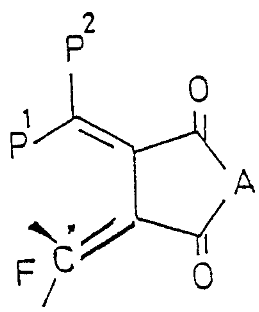

(IIIa<u>A</u>)

It will be appreciated that whether or not a particular compound of formula (IIIa) can exist in separate helical forms is dependent on the identities of $P^1$ and ⌐ and particularly their relative sizes. However even if a given compound (IIIa) does not exist in separate helical forms, separate spiro forms may arise in the cyclised structure formed when the compound is exposed to electromagnetic radiation of the characteristic wavelength.

It will be appreciated that for a compound of given formula the total number of isomers can thus be eight.

The compounds of the invention of formulae (IIIa) and (IIIb) can exist in a further set of isomeric forms as a result of geometric isomerism about the double bond linking the group

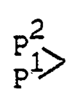

to the remainder of the molecule. The photoactive compounds of formulae (IIIa) and (IIIb)

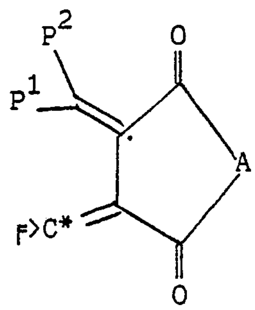

(IIIa)

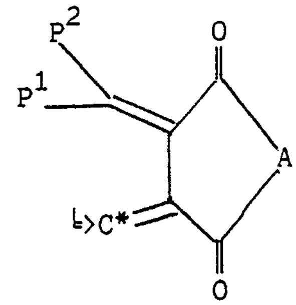

(IIIb)

are thus geometric isomers of the non-photoactive compounds of formulae (IIIc) and (IIId)

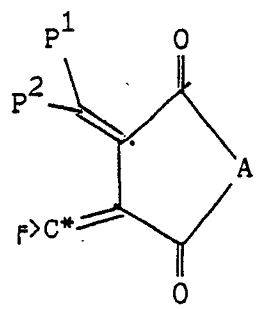

(IIIc)

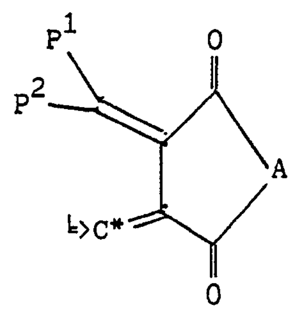

(IIId)

The synthetic procedures described below can produce mixtures of geometric isomers, including not only the desired photoactive compounds of formulae (IIIa) and (IIIb), but also the non-photoactive compounds of formulae (IIIc) and (IIId). In certain instances, steric factors may result in compounds of formulae (IIIc) and (IIId) predominating.

However, compounds of formulae (IIIc) and (IIId) may be converted into compounds of formula (IIIa) and (IIIb) by exposure to ultraviolet light. Exposure of compounds of formulae (IIIa) and (IIIb) to ultraviolet light will also result in their conversion to compounds of formulae (IIIc) and (IIId), i.e. the reaction is reversible and but for the fact that the compounds of formula (IIIa) and (IIIb) are photoactive, an equilibrium mixture of compounds of formulae (IIIa-d) would be formed. The fact that the compounds of formula (IIIa) and (IIIb) are photoactive, however, permits exposure to ultraviolet light to be used to convert compounds of formula (IIIc) and (IIId) quantitatively to compounds of formula (IIIa) and (IIIb). As explained below, such exposure should in general be in the substantial absence of white light or monochromatic light in the visible region.

As indicated above, the compounds of formulae (IIIa) and (IIIb) are photoactive, but the compounds of formulae (IIIc) and (IIId), are not. That is to say only compounds of formulae (IIIa) and (IIIb) are capable of undergoing a structural change when subjected to electromagnetic radiation of a selected wavelength or wavelengths and undergo a reversal to their original form when subjected to thermal energy or to electromagnetic radiation of another wavelength.

Desired photoactive compounds of formulae (IIIa) and (IIIb) may thus be produced by a procedure, which makes use of the differences in the properties of the compounds of formulae (IIIa-d), particularly the property that compounds of formulae (IIIa) and (IIIb) are photoactive, but compounds of formulae (IIIc) and (IIId) are not.

This procedure comprises irradiating a compound of formula (IIIc) or (IIId) with electromagnetic radiation, e.g. 366 nm U.V. capable of converting said compound to its photoactive geometric isomer of formula (IIIa) or (IIIb). This electromagnetic radiation, e.g. 366 nm U.V. additionally causes compounds of formula (IIIa) or (IIIb) to undergo a photoactive structural change.

In summary the conversion caused by the exposure of the compounds of formula (IIIc) and (IIId) to ultraviolet light so as to form compounds of formula (IIIa) and (IIIb) is reversible and as indicated above, in itself results in an equilibrium mixture of compounds of formulae (IIIa-d) being formed. However exposure of

so-formed compounds of formulae (IIIa) and (IIIb) to the same electromagnetic radiation can result in the net production of the product of the photoactive change, because the reverse process of the photoactive change is negligible under the radiation conditions e.g. at the wavelength of 366 nm. The photoactive change may, of course, be reversed by subjecting the product of the photoactive change to, for example, electromagnetic radiation of a wavelength other than that causing the photoactive change, e.g. white light or monochromatic light in the visible range.

The above reactions may be represented by the following:

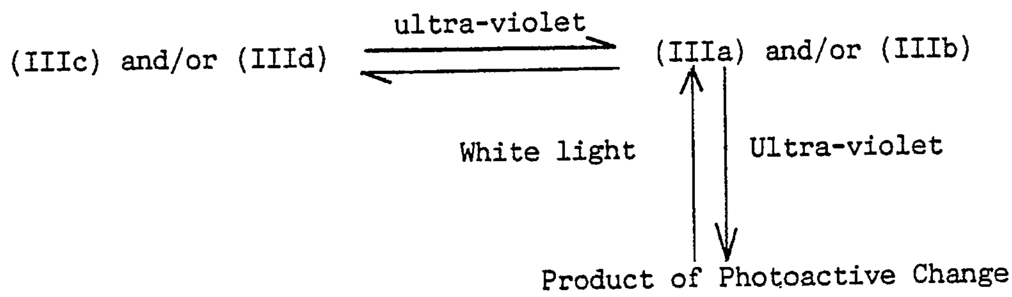


In a preferred method of operation, a compound of formula (IIIc) or (IIId) is exposed to near U.V. (300-400nm) light. The result of such exposure is the net production of the compound or compounds referred to above as the "Product of Photoactive Change".

As indicated above, however, this "Product of Photoactive Change" may be converted back to the desired photoactive compounds of formulae (IIIa) and (IIIb) by subjecting it to electromagnetic radiation of an appropriate wavelength, e.g. white light or light in the visible region absorbed by the product of photoactive change.

Since compounds of formula (IIIa) to (IIId) are near colourless, they do not absorb white light or light in the visible region and therefore no photoactive change is undergone by compounds of formula (IIIa) to (IIId) on exposure to white light.

Thus the following overall sequence of reactions may be used to obtain desired photoactive compounds of formulae (IIIa) and (IIIb) in high yields.

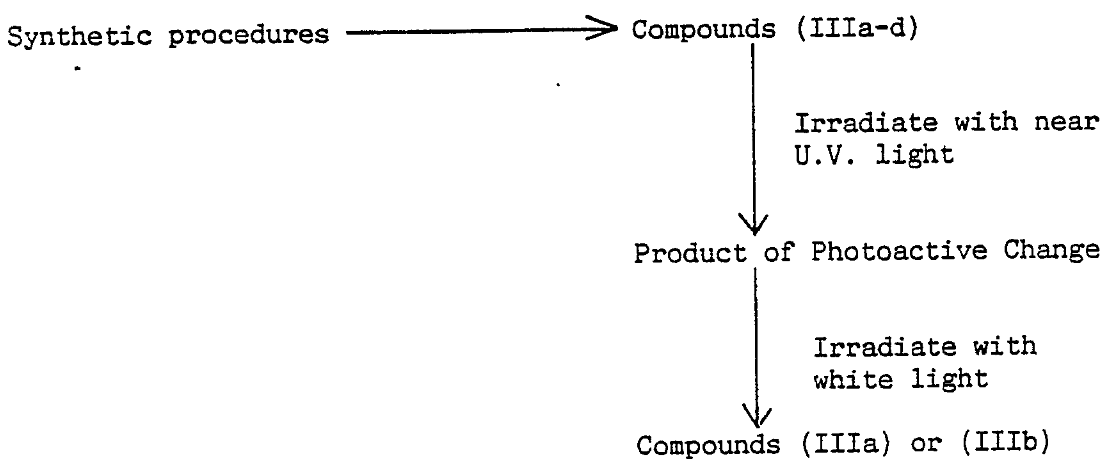


General Methods of Synthesis

The compounds of the invention of formula (IIIa) and (IIIb) and the non-photoactive geometric isomers of formula (IIIc) and (IIId) may be prepared by the Stobbe condensation from a lower dialkyl succinates, e.g. diethyl succinate.

Advantageously a modification to conventional Stobbe reaction conditions is employed which will be discussed in further detail below and forms a further aspect of the invention.

Various sequences of reaction may be used, e.g. the sequences referred to as "Sequence 1" and "Sequence 2" below.

The production of compounds of formulae (IIIa-d) by Sequence 1 is as follows:

Sequence 1

In a first step a ketone (VI) is reacted with a di-lower alkyl succinate (VII, R = lower $C_{1-4}$ alkyl)

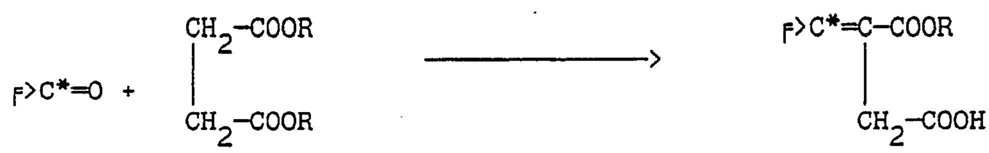

(VI) (VII) (VIII)

to yield condensation product VIII. The reaction is carried out in the presence of a basic catalyst e.g. as described in more detail below.

Condensation product VIII is esterified to form the diester (IX, R = lower alkyl) which is then reacted with a ketone $P^1COP^2$

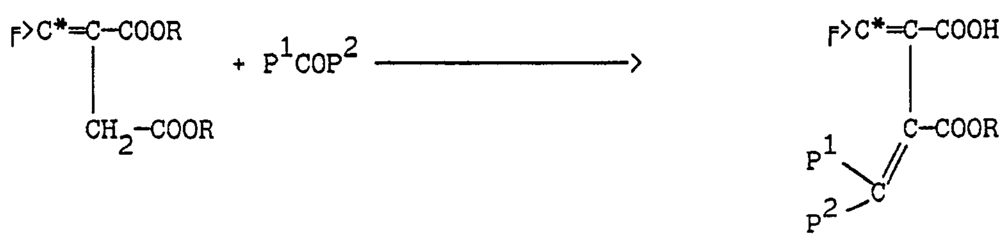

(IX) (X)

Half-ester X is hydrolysed to form the corresponding dicarboxylic acid and then converted into the desired compound of formula III by treatment with a dehydrating agent (e.g. an acyl halide) to form a compound in which A is oxygen, which in turn can be converted into a compound in which A represents $NR^1$ by reacting with an amine $H_2NR^1$.

The production of compounds according to the invention by Sequence 2 will now be described.

Sequence 2

In a first step, a ketone $P^1COP^2$ may be reacted with a di-lower alkyl succinate (VII, R = lower $C_{1-4}$ alkyl)

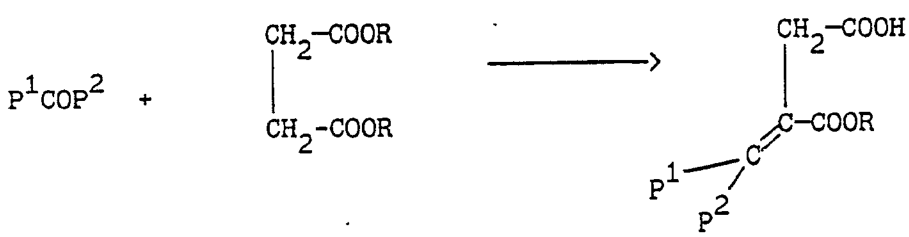

(VII) (XI)

to yield a condensation product XI. The reaction is carried out in the presence of a basic catalyst, e.g. as

described in more detail below.

Condensation product XI is esterified to form the diester (XII, R = lower alkyl) which is then reacted with a ketone (VI).

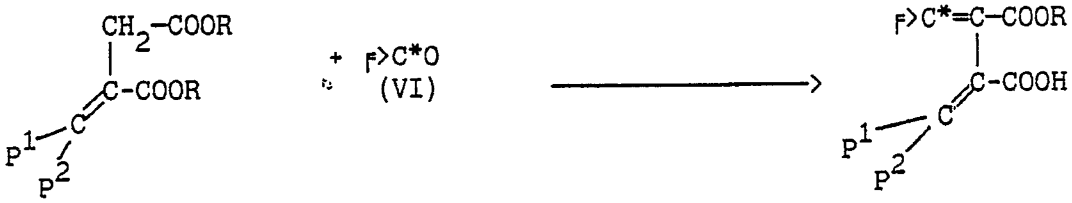

(VI)

(XIII)

(XII)

Half-ester (XIII) is hydrolysed to form the corresponding dicarboxylic acid and then converted into the desired compounds IIIa and IIIb as described above.

It will be appreciated that in each of Sequences 1 and 2 depicted above the products of the Stobbe condensations VIII, X, XI and XIII may be produced in plural isomeric forms.

Thus product VIII may be produced as a mixture of E and Z isomers.

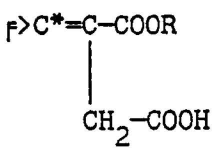

(VIIa)

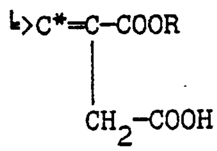

(VIIb)

Product X may be produced as a mixture of E,E, E,Z, Z,Z and Z,E isomers.

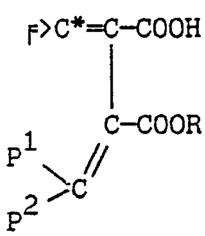

(Xa)

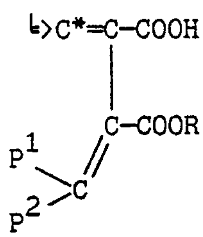

(Xb)

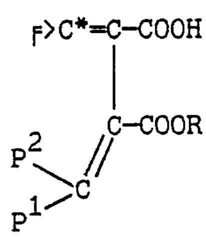

(Xa')

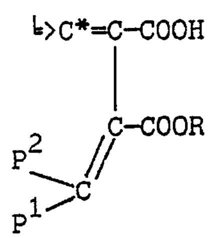

(Xb')

Product XI may be produced as a mixture of E and Z isomers.

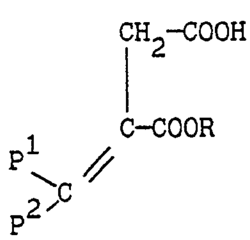

(XIa)

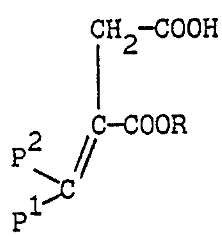

(XIb)

Product XIII may be produced as a mixture of E,E, E,Z, Z,Z and Z,E isomers

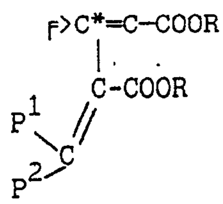 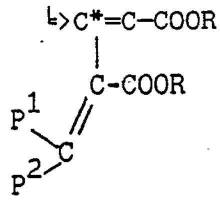 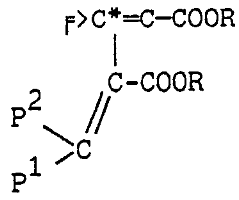 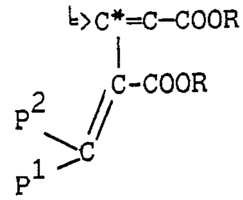

(XIIIa) (XIIIb) (XIIIa') (XIIIb')

For the production of the class of compounds in which F>C* represents one of structures Va-Vd set forth above, a corresponding ketone of formula VIa′-VId′ would be used. The formulae (and where available, the trivial names) of these ketones are as follows:

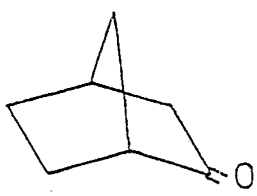

VIa'
bicyclo[2.2.1]heptan-2-one
(norcamphor)

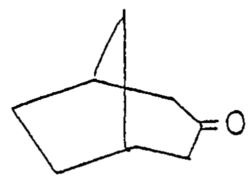

VIb'
bicyclo[3.2.1]octan-3-one

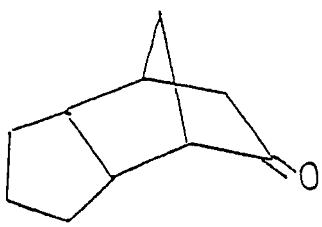

VIc'
tricyclodeca[5.2.1.0$^{2,6}$]-8-one
(tetrahydro-dicyclopentadienone)

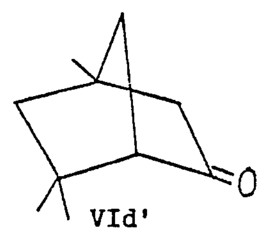

VId'
4,7,7-trimethyl-bicyclo[2.2.1]-heptan-2-one
(isofenchone)

Ketones of related formula include camphor (VIa′, but having methyl groups in the 1,7,7-positions which is available as the racemate (produced synthetically) and as the d-enantiomer (obtained from natural sources). Similarly fenchone isolated from natural sources is available in optically active form. It will be appreciated that the use of single enantiomers of ketones $P^1COP^2$ enables optically active forms of compounds of formula III to be obtained without the need to resolve starting materials, intermediates or products into separate enantiomers.

Conventionally, in carrying out a Stobbe condensation in which a ketone is condensed with a dialkyl succinate, the reaction is generally carried out in ethanol or t-butanol, using sodium ethoxide or potassium t-butoxide as catalyst respectively. Alternatively the reaction has been carried out in toluene, using sodium hydride plus a trace of ethanol, as catalyst.

Yields obtainable with existing reaction media and catalysts can be low, particularly when the reactants are sterically hindered. We have now unexpectedly found that a particular catalyst/reaction medium combination gives improved high yields.

Thus according to this further aspect of the invention there is provided a process for producing a condensation product between a lower alkyl ester of succinic acid (or a methylene derivative of succinic acid in which at least one of the hydrogen atoms of the methylene groups is replaced by a substituent which does not interfere with the reaction) and a ketone, which comprises reacting said ester and said ketone in the presence of an aromatic hydrocarbon, preferably toluene, and an alkali metal alkoxide,

preferably potassium t-butoxide. According to a preferred embodiment of the process of the invention (Sequence 1) there is provided a process for producing a compound of formula

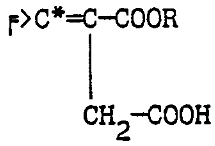

(VIII)

wherein R and F>C* are as defined above which comprises reacting a di-lower alkyl succinate of general formula

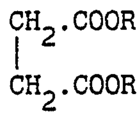

(VII)

wherein R is as defined above with a ketone of formula
F>C* = CO (XIII)
wherein F>C* is as defined above, characterised in that the reaction is carried out in a reaction medium comprising toluene and in the presence of potassium t-butoxide as catalyst.

The resulting compound of formula (VIII) may then be esterified to form a compound of formula IX

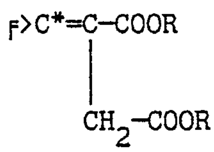

(IX)

and reacted with a ketone of formula $P^1COP^2$, wherein $P^1$ and $P^2$ are as defined above to form a compound of formula (IIIa). According to a preferred embodiment of the process of the invention (Sequence 2) there is provided a process for producing a compound of formula

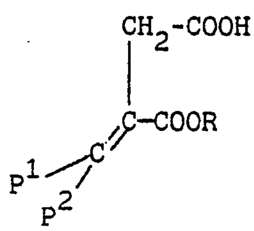

(XI)

wherein R, $P^1$ and $P^2$ are as defined above which comprises reacting a di-lower alkyl succinate of general formula

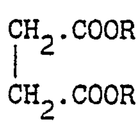

(VII)

wherein R is as defined above with a ketone of formula $P^1.P^2CO$ (XIII)
wherein $P^1$ and $P^2$ are as defined above, characterised in that the reaction is carried out in a reaction medium comprising toluene and in the presence of potassium t-butoxide as catalyst.

The resulting compound of formula (VIII) may then be esterified to form a compound of formula IX

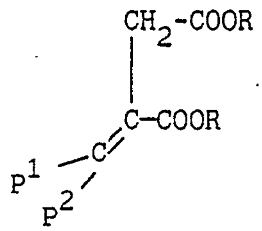

and reacted with a ketone of formula ꟷ>C*O (XIII), to form a compound of formula (IIIa).

The production of compounds according to the invention will now be described by way of example, with particular reference to the attached Reaction Schemes and Formulae Drawings.

## Example 1

**Production of Photoactive Fulgides (Formulae IIIa and IIIb; ꟷ>C* = bicyclo[2.2.1]heptan-6-ylidene; $P^1$ = 2,5-dimethyl-furan-3-yl; $P_2$ = methyl; A = O) By Condensation of Norcamphor (bicyclo[2.2.1]-heptan-6-one (1)) with Diethyl Succinate and Subsequent Reaction With 2,5-Dimethyl-3-acetyl-furan**

1.1 Preparation of ethyl E- and Z-bicyclo[2.2.1]heptan-6-ylidenesuccinate (2, R = H) and (3, R = H)

Norcamphor (1) (100 g, 0.9 mole) and diethyl succinate (174 g, 1 mole) were dissolved in dry toluene (150 ml) and the solution was added dropwise with stirring to a suspension of potassium t-butoxide (122 g, 1 mole) in dry toluene (250 ml) initially at room temperature. The reaction was exothermic and the reaction mixture gradually warmed up to 60 °C and the potassium t-butoxide dissolved. The reaction mixture was allowed to stir for 3 hours. Water (1 l) and ether (250 ml) were added and the mixture was carefully shaken in a separator. The potassium salt of the required half ester was extracted into the aqueous layer which was separated. The alkaline aqueous layer was acidified with concentrated hydrochloric acid. The liberated half ester crystallised and was filtered off. The yield of the 1:1 mixture of E- and Z-half esters (2,3, R = H) was 155 g (76%). Structure and stereochemistry was established by n.m.r. spectroscopy.

1.2 Preparation of diethyl E- and Z-bicyclo[2.2.1]hepta-6-ylidenesuccinate (2, R = Et) and (3, R = Et).

The mixed half esters (155 g) were boiled with ethanol (75 parts by volume), toluene (225 parts by volume) and conc. hydrochloric acid (2 parts by volume) and the water azeotroped off using a Dean and Stark apparatus. Unchanged half ester was extracted with sodium carbonate solution, the organic layer was extracted with sodium carbonate solution, the organic layer was dried (magnesiun sulphate), filtered and the solvent removed. The diester was obtained as a colourless oil b.p. 120-130 °C at 0.1 mm Hg and was a 1:1 mixture of the E- and Z-esters.

1.3. Preparation of photochromic fulgides (4) and (5).

The 1:1 mixture of diesters (21.5 g, 0.1 mole) and 2,5-dimethyl-3-acetyl furan (13.8 g, 0.1 mole) were dissolved in toluene (50 ml) and added dropwise with stirring over 1 hour to an ice-cold suspension of potassium t-butoxide (11.2 g, 0.1 mole) in toluene (150 ml). When the addition was complete, the mixture was stirred for a further hour. The mixture was poured into ice-water (200 ml) and the aqueous layer was separated and acidified with 5M hydrochloric acid. The liberated half-esters were extracted with ether (2 x 150 ml), the ether extract was dried over anhydrous magnesium sulphate and solvent removed. The residual half esters (27 g) were hydrolysed by boiling with 10% ethanolic potassium hydroxide for 2 hours. Ethanol was removed and the dipotassium salt of the diacid was dissolved in water and acidified with 2M hydrochloric acid.

The liberated diacid was extracted with ether (2 x 150 ml), dried over anhydrous magnesium sulphate,

filtered and the solvent removed.

1.4 The residual oil was treated with acetyl chloride (100 ml) and the solvent removed. The residue was treated with a small amount of isopropanol which caused some solid to separate, which was twice crystallised from petroleum (b.p. 60-80° C) to give colourless crystals of a mixture of the fulgides. The fulgides show a marked colour change to red on exposure to 366 nm radiation which bleaches on exposure to white light.

Example 2

**Production of Photochromic Fulgides (Formulae IIIa and IIIb; F >C* = tricyclodeca[5.2.1.O$^{2,6}$]-8-ylidine; $P^1$ = 2,5-dimethylfuran-3-yl; $P^2$ = methyl; A = O) By Condensation of Tricyclodeca-[5.2.1.O$^{2,6}$]-8-one with Diethyl Succinate and Subsequent Reaction With 2,5-Dimethyl-3-Acetyl Furan**

An analogous sequence of reactions was carried out using tricyclodeca[5.2.1.O$^{2,6}$]-8-one (6) in place of norcamphor (1). The resulting mixture of fulgides shows photochromic properties similar to the photochromic properties of the fulgide mixture (4 and 5) described above.

Example 3

**Production of Photochromic Fulgides (Formulae IIIa and IIIb; F>C* = bicyclooctan [3.2.1]-3-ylidine, $P^1$ = 2,5-dimethyl-furan-3-yl, $P^2$ = methyl; A = O) By Condensation of Tricyclodeca[5.2.1.O$^{2,6}$]8-one with Diethyl Succinate and Subsequent Reaction With 2,5-Dimethyl-3-Acetyl Furan**

Bicyclooctan[3.2.1]-3-one (7) was prepared and condensed with diethyl succinate as described above. The half ester (8) was obtained as colourless crystals from toluene and petroleum in 58% yield.

Example 4

**Production of Photoactive Fulgides (Formulae IIIa and IIIb; F >C* = bicyclo[2.2.1]heptan-6-ylidine; $P^1$ = 2,5-dimethyl-furan-3-yl, $P_2$ = methyl; A = O) By Condensation of Norcamphor (bicyclo[2.2.1]-heptan-6-one (1)) with Dimethyl Succinate and Subsequent Reaction With 2,5-Dimethyl-3-acetyl-furan**

This example is a modification of the procedure of Example 1, but utilising dimethyl succinate as starting material.

4.1 Norcamphor (1) (100 g), dimethyl succinate (140 g), potassium t-butoxide (105 g) in toluene (500 $cm^3$), gave a 89% yield of a 1:1 mixture of half esters (9; R = H) and (10; R = Me) (174 g).

4.2 The 1:1 mixture of half esters (9; R = H) and (10; R = H) (25 g, 0.11 mole), ethanol (13.8 g; 0.3 mole), and toluene-p-sulphonic acid (1 g) were dissolved in toluene (100 $cm^3$) and the solution refluxed for 4 hours, using an Dean and Stark apparatus to remove water. After work-up and removal of solvent, the residual oil was distilled (b.p. 160-180° C at 0.8 mm) to give the mixture of esters (9; R = Et) and (10; R = Et) in 90% yield (25 g).

Compared to Example 1, the overall yield for the first synthetic intermediate was increased from 44% to 80%, i.e. the yield has been doubled.

4.3 Condensation of 3-acetyl-2,5-dimethylfuran with the 1:1 mixture of Z and E-dimethyl esters (9 and 10; R = Me). A 1:1 mixture of the Z and E-dimethyl esters (9 and 10; R = Me) (56 g, 0.26 mole) and 2,5-dimethyl-3-acetyl furan (41.4 g, 0.29 mole) in toluene (100 $cm^3$) was added dropwise with stirring to potassium t-butoxide (33 g, 0.3 mole) in toluene (300 $cm^3$) cooled to 0° C in an icebath.

When the addition was complete, the reaction mixture was allowed to warm up to room temperature and then poured into water (750 $cm^3$). The aqueous layer was separated and acidified with concentrated hydrochloric acid. The liberated half-esters were extracted with ether (2 x 300 $cm^3$), dried over anhydrous magnesium sulphate, filtered and the solvent removed. A solution of potassium hydroxide (20 g) in ethanol

($200\ cm^3$) was added to the residual oil and the mixture was boiled for 3 hours. Most of the ethanol was distilled off and 2-propanol ($150\ cm^3$) was added.

The warm solution was left overnight and the solid dipotassium salt was filtered off, dissolved in water (1 l) and acidified with concentrated hydrochloric acid.

The liberated oil was extracted with ether ($3 \times 400\ cm^3$) and dried as before, filtered, and most of the solvent removed. Acetyl chloride ($200\ cm^3$) was added dropwise to the solution over 1 hour and stirred for a further hour and the solvent removed. Petroleum (b.p. 60-80°C) ($400\ cm^3$) was added, together with anhydrous sodium carbonate (5 g) and charcoal (1 g) and the mixture was boiled for 10 minutes, and filtered hot. On cooling, a 1:1 mixture of the E,Z- and E,E-fulgides (15; X=O) and (16; X=O) separated as pale cream needles (12 g) (16% yield).

Example 5

**Production of Photoactive Fulgides (Formulae IIIa and IIIb; F>C* = bicyclo[2.2.1]heptan-6-ylidine; $P^1$ = 2,5-dimethyl-thiophen -3-yl, $P_2$ = methyl; A = O) By Reaction of Norcamphor (bicyclo[2.2.1]-heptan-6-one (1)) with Product of Condensing Dimethyl Succinate and 2,5-Dimethyl-3-acetyl-thiophen**

A 1:1 mixture of the dimethyl esters (9 and 10; R=Me) (30 g, 0.14 mole) and 2,5-dimethyl-3acetylthiophene (25 g, 0.16 mole) in dry toluene was added with stirring to a suspension of potassium t-butoxide (17.2 g) in dry toluene ($200\ cm^3$) cooled in an icebath. The cold mixture was stirred (4 h), allowed to warm to room temperature and poured onto water ($250\ cm^3$). The aqueous layer was acidified with concentrated hydrochloric acid and the liberated oily half esters were extracted with ether and hydrolysed by boiling with 20% ethanolic potassium hydroxide for 4 hours. When half of the ethanol had distilled off, 2-propanol ($200\ cm^3$) was added, and the warm solution was left to crystallise overnight. The dipotassium salt was filtered off, dissolved in water (1 l) and acidified with concentrated hydrochloric acid. On addition of ether (1 l) the diacid (1.3 g) separated and was filtered off. Work up of the ether extract gave a gum to which acetyl chloride ($200\ cm^3$) was added dropwise over a period of 2 hours and then stirred for a further 2 hours. Removal of solvent left a colourless solid, which was recrystallised from dichloromethane and petroleum (b.p. 60-80°C) (1:4) to give the E,Z-fulgide (15; X=S) (5.3 g, 14%) followed by a second crop of crystals which was a mixture of the E,Z and E,E-fulgides (15; X=S) and (16; X=S).

Example 6

**Production of Photoactive Fulgides (Formulae IIIa and IIIb; F>C* = bicyclo[2.2.1]heptan-6-ylidine; $P^1$ = 2-methyl-5-phenylfuran-3-yl, $P_2$ = methyl; A = O) By Reaction of Condensation Product of Norcamphor (bicyclo[2.2.1]-heptan-6-one (1)) and Dimethyl Succinate With 3-Acetyl-2-methyl-5-phenylfuran**

Condensation of 3-acetyl-2-methyl-5-phenylfuran (14) with 1:1 mixture of Z- and E-dimethyl esters (9 and 10; R=Me).

A 1:1 mixture of the norcamphor methyl esters (9 and 10; R=Me) (20 g, 0.09 mole) and 3-acetyl-2-methyl-5-phenylfuran (15 g 0.08 mole) in dry toluene ($100\ cm^3$) was added dropwise with stirring to a suspension of potassium t-butoxide (12 g) in dry toluene cooled to 0°C in an icebath. The mixture was stirred for 5 hours, allowed to warm up to room temperature, poured into water ($500\ cm^3$), and acidified with conc. hydrochloric acid.

The liberated half-esters were separated and boiled with 10% ethanolic potassium hydroxide. On cooling and standing, the dipotassium salt of the diacid separated and was filtered off. The salt was dissolved in water ($500\ cm^3$), acidified with concentrated hydrochloric acid, and treated with ether. The diacid (2.7 g) separated and was filtered off. The acid solution was extracted with ether ($2 \times 200\ cm^3$) and the diacid separated as before.

Acetyl chloride ($150\ cm^3$) was added dropwise to the diacid over 3 hours at room temperature. Solvent

was removed and the residue was dissolved in chloroform (100 $cm^3$), washed with potassium carbonate solution, dried over magnesium, sulphate, filtered, and the solvent removed. The residue was dissolved in 2-propanol (50 $cm^3$). On standing, a mixture of the two photochromic (E,Z) and (E,E) fulgides (17) and (18) crystallised out (8.1 g) which were separated by repeated recrystallisation and separation of the two different types of crystals by hand.

Example 7

Condensation of Bicyclo[3.2.1]octan-3-one With Diethyl Succinate

7.1 In a modified procedure, a solution of bicyclo[3.2.1]octan-3-one (VIb′) (5 g 0.04 mole) and diethyl succinate (7 g, 0.04 mole) dissolved in toluene (10 $cm^3$), was added dropwise with stirring to a suspension of potassium t-butoxide (5.38 g, 10% excess) in toluene (50 $cm^3$). Work up as before gave the half ester (23; R = H) in 90% yeild.

7.2 Re-esterification Process

The half-ester (23; R = H) (5 g, 0.021 mole) and methanol (1.36 g, 0.42 mole) was dissolved in dichloromethane (5 g) and N,N′-dicyclohexycarbodiimide (4.34 g, 0.021 mole) in dichloromethane (10 g) was added dropwise with stirring. The reaction mixture was boiled overnight. The precipitate of dicyclohexylurea was filtered off, the filtrate was washed first with 5M hydrochloric acid to remove excess reagent, and then with dilute sodium hydroxide solution to remove unreacted acid. The solution was dried over magnesium sulphate, filtered, and the solvent removed. The crude dimethyl ester (23; R = Me) was distilled under high vacuum. Yield 27% (b.p. 190-210 °C at 0.5 mm.

An improved esterification process was developed. The half ester (23; R = H) and ethanol and p-toluenesulphonic acid in toluene was refluxed using a Dean and Stark Apparatus to remove water. The yield of the diester (23; R = Et) using this simpler procedure was over 60%.

7.3 Synthesis of Photoactive Fulgides (Formulae IIIa and IIIb; F>C* = bicyclo[3.2.1]octan-3-ylidine; $P^1$ = 2,5-dimethyfuran-3-yl; $P^2$ = methyl; A = O)

Diester (23) (5 g, 18 mmole) and 3-acetyl-2,5-dimethylfuran (2.5 g, 18 mole) were condensed by the Stobbe procedure using potassium t-butoxide (2.33 g) in toluene (85 $cm^3$). The half ester(s) were obtained in 72% yield, and were hydrolysed by boiling with 10% ethanolic potassium hydroxide (100 $cm^3$) overnight. The solution was concentrated and the potassium salt of the diacid separated, filtered off, dissolved in a small amount of water, and acidified carefully with hydrochloric acid. The liberated diacid was extracted with ether. The extract was dried ($MgSO_4$), filtered and the solvent removed leaving the crude diacid (3.15 g, 85% yield).

The diacid (1.13 g 3.3 mmole) was dissolved in ether and treated with acetyl chloride. Solvent was removed leaving the crude fulgide which was extracted into petroleum (b.p. 80-100 °C) the solution was concentrated and crystals of the fulgide separated. Comparison of the crystals prepared in this manner with the sample prepared by the alternative procedure described in Example 8.3, using 13C and 1 H n.m.r. spectroscopy and u.v./visible spectroscopy indicated that the samples are different isomers of Compound (26). Comparison of the spectra with that of a sample obtained previously indicates that the previous sample is the Z-isomer and the sample obtained in this experiment is the (E)-isomer (16, X = O).

This does not necessarily mean that only one isomer is formed in each synthetic procedure but that for each procedure only one isomer was isolated.

Example 8

**Production of Photochromic Fulgides (Formulae IIIa and IIIb; F>C* = bicyclo[3.2.1]octan-3-ylidine; $P^1$ = 2,5-dimethyl- furan-3-yl, $P^2$ = methyl; A = O)**

This example illustrates an alternative procedure with the order of the two condensation reactions reversed. In this procedure 3-acetyl-2,5-dimethylfuran is reacted with diethyl succinate first and finally condense with bicyclo [3.2.1]octan-3-one.

8.1 Preparation of E- and Z-ethyl-2,5-dimethyl-3-furyl-ethylidenesuccinates.

3-Acetyl-2,5-dimethylfuran (10 g; 0.0723 mole) and diethyl succinate (12.6 g; 0.0723 mole) were dissolved in toluene (50 $cm^3$) and added dropwise with stirring to a suspension of potassium t-butoxide (9.2 g; 10% excess) in toluene (150 $cm^3$) warmed initially to 65°C. The reaction mixture was stirred at room temperature overnight. The reaction mixture was poured into water and extracted until the deep red colour of the toluene layer discharged. The aqueous layer was acidified with conc. hydrochloric acid and the liberated oily half-esters (24 and 25; R = H) extracted with ether. The ether layer was dried over magnesium sulphate, filtered, and the solvent removed leaving a crude yield of half-esters (95%).

8.2. E- and Z-Diethyl 2,5-diethyl-3-furylethylidenesuccinates

The crude half esters (24 and 25; R = H) 19 g were re-esterified by boiling with ethanol (9.3 g) and with p-toluenesulphonic acid (0.24 g). The yield of diesters (24 and 25; R = Et) (b.p. 120°C at 10.5 mm) was low but the unreacted half esters (24 and 25; R = H) were recovered quantitatively.

8.3 Condensation of the succinic esters (24 and 25; R = Et) with bicyclo[3.2.1]octan-3-one.

A mixture of the diesters (24 and 25; R = Et) (5 g, 0.017 mole) and bicyclo[3.2.1]octan-3-one (2.11 g, 0.017 mole) in toluene (10 $cm^3$) was added dropwise with stirring to a suspension of potassium t-butoxide (2.29 g, 10% excess) in toluene (50 $cm^3$) at 5°C. Work up as before gave a 77% yield of half esters (26 and 27; R = H). The half esters (2 g, 5.4 mmole) were hydrolysed by boiling with 10% ethanolic potassium hydroxide (25 $cm^3$). Ethanol was removed under reduced pressure. The potassium salts were dissolved in a small amount of water and acidified with hydrochloric acid. The precipitated diacids were extracted into ether, which was dried over magnesium sulphate, filtered and solvent evaporated. The residual diacids (1.44 g) were dissolved in water (50 $cm^3$) and acetyl chloride (10 $cm^3$) was added. Solvent was removed under reduced pressure and the residue was treated with petroleum (b.p. 80-100°C). A small amount of sodium carbonate was added to remove trace amounts of acid and charcoal was added to remove coloured impurities. The solution was heated to boiling and filtered. On cooling, crystals separated.

Their solutions are photochromic, undergoing a colourless to red colour change which is bleached by white light, demonstrating that during the course of these reactions, the endocyclic double bond has migrated back to give the required (E)-fulgide with the exocyclic double bond.

Example 9

**Production of Photochromic Fulgides (Formulae IIIa and IIIb; F>C* = tricyclodeca[5.2.1.$O^{2,6}$]-8-ylidine; $P^1$ = 2,5-dimethylfuran-3-yl; $P^2$ = methyl; A = O) By Condensation of Tricyclodeca-[5.2.1.$O^{2,6}$]-8-one with Dimethyl Succinate and Subsequent Reaction With 2,5-Dimethyl-3-Acetyl Furan**

Condensation of Tricyclodeca[5.2.1.$O^{2,6}$]-8-one (Tetrahydro-dicyclopentadienone (VIc′)) with Dimethyl Succinate

9.1 A solution of tetrahydro-dicyclopentadienone (300 g, 2 mole) and dimethyl succinate (325 g, 2.05 mole) in dry tetrahydrofuran (200 $cm^3$) was added slowly to a stirred solution of potassium t-butoxide (240 g, 2.1 mole) dissolved in dry tetrahydrofuran (1.5 l). When the addition was complete, the reaction mixture was stirred at room temperature for a further 4 hours. Water (1 l) was added and the tetrahydrofuran removed on a rotatory evaporator under reduced pressure. Water (1 l) was added and the solution was extracted with ether (0.5 l). The aqueous layer was separated and acidified with concentrated hydrochloric

acid and the liberated oil extracted into ether (3 x 0.5 l). The combined ether extracts were dried ($MgSO_4$), filtered and solvent removed. Methanol (750 $cm^3$) was added to the residue which was heated to boiling and thionyl chloride (125 $cm^3$) was added slowly (3 h). Solvent was removed and the residue dissolved in ether (1.5 l) and extracted with 1M sodium hydroxide solution to remove unreacted acid. The ether extract was dried ($MgSO_4$), filtered and the ether removed. The residual oil was distilled under vacuum to give a mixture of the pure dimethyl esters (462 g, 85% yield) b.p. 135-158 °C at 0.025 mm.

9.2 A mixture of the above dimethyl esters (60 g 0.2 mole) and 3-acetyl-dimethylfuran (30 g, 0.2 mole) in dry toluene were added dropwise (1.5 h) to a stirred suspension of potassium t-butoxide (27 g, 0.22 mole) in dry toluene (300 $cm^3$) cooled to below 5 °C in an ice/salt bath. The mixture was stirred (2 h) and water (0.5 l) was added. The toluene layer was separated. The aqueous layer was acidified with concentrated hydrochloric acid and the liberated half-esters were extracted with ether (2 x 400 $cm^3$). The ether extract was dried ($MgSO_4$), filtered and the solvent removed and the residual half-esters were hydrolysed by boiling (4 h) with 10% ethanolic potassium hydroxide (200 $cm^3$). After standing (2 days), the dipotassium salt was filtered off, dissolved in water (400 $cm^3$) and carefully acidified with concentrated hydrochloric acid.

The liberated diacids were extracted with ether, dried ($MgSO_4$), filtered and the solvent removed. The residual diacids were flash chromatographed on silica gel (100 g) using petroleum (b.p. 60-80 °C) as eluant. Solvent was removed leaving the only acids (5 g) which were dissolved in ether (200 $cm^3$) and acetyl chloride (25 $cm^3$) was added dropwise with stirring. After 2 hours, the solvent was removed and the residual fulgides were purified by chromatography on silica gel using petroleum as eluant. The fractions were concentrated and on standing a mixture of the two photochromic fulgides( a mixture of E,Z- and E,E-isomers) separated and were recrystallised from petroleum to give the pure E,E- and E,Z-isomers (450 mg). The relatively poor overall yield is attributed to the high solubility of the product in organic solvents, making it difficult to crystallise.

Example 10

**Production of Photochromic Fulgides (Formula IIIa and IIIb; ⌐>C* = tricyclodeca[5.2.1.O$^{2,6}$]-8-ylidine; $P^1$ = 2,5-dimethyl-thiophen-3-yl; $P^2$ = methyl; A = O) By Condensation of Dimethyl Esters of Example 9.1 With 3-Acetyl-Dimethylthiophene**

10.1 A mixture of the dimethyl esters (60 g, 0.2 mole) obtained as in Example 9.1 and 3-acetyl-dimethylthiophene (32 g, 0.2 mole) in dry toluene were added dropwise (1.5 h) to a stirred suspension of potassium t-butoxide (27 g, 0.22 mole) in dry tetrahydrofuran (450 $cm^3$) cooled to below 5 °C in an ice/salt bath. The mixture was stirred (2 h) and water (1 l) was added and tetrahydrofuran removed under reduced pressure. The aqueous layer was extracted with ether and then acidified with concentrated hydrochloric acid. The liberated half-esters were extracted with ether (2 x 500 $cm^3$). The ether extract was dried ($MgSO_4$), filtered and the solvent removed and the residual half-esters were hydrolysed by boiling (4 h) with 10% ethanolic potassium hydroxide (500 $cm^3$). After standing overnight, the dipotassium salt was filtered off, dissolved in water (1 l) and carefully acidified with concentrated hydrochloric acid.

On addition of ether (500 $cm^3$), a colourless solid separated (2.7 g) which were dissolved in acetone (25 $cm^3$) and treated with acetyl chloride (10 $cm^3$). Removal of solvent gave a colourless solid which, on recrystallisation from a 1:5 mixture of dichloromethane and petroleum, gave a non-photochromic fulgide as pale yellow needles (1.7 g), tentatively assigned the Z-configuration. The aqueous layer was re-extracted with ether (500 $cm^3$), and the combined ether layers were dried ($MgSO_4$) and filtered.

Acetyl chloride (200 $cm^3$) was added dropwise to the filtrate over 1 hour and the reaction mixture was left to stand overnight. Removal of solvent left a product which was flash chromatographed on silica gel using petroleum as eluant. The chromatographic fraction, on concentration, gave a further crop of the Z-isomer (2.3 g).

10.2 Photochemical Z-E isomerisation

The Z isomer from 10.1 (2 g) in toluene (1.5 l) was irradiated (24 h) with unfiltered light from a medium pressure mercury lamp contained in an immersion glass thimble (Hanovia photoreactor). Toluene was removed and the residue was flash chromatographed on silica gel using petroleum as eluant. The solution

was concentrated and gave, on standing, crystals of the E-isomer.

Example 11

**Production of Photoactive Fulgides (Formulae IIIa and IIIb; F>C* = bicyclo[2.2.1]heptan-6-ylidine; $P^1$ = 5-p-fluorophenyl-2-methyl-furan-2-yl; $P_2$ = methyl; A = O) By Condensation of Norcamphor (bicyclo[2.2.1]heptan-6-one (1)) with Diethyl Succinate and Subsequent Reaction With 3-Acetyl-p-fluorophenyl-2-methyl-furan**

A solution of 3-acetyl-5-p-fluorophenyl-2-methylfuran (11 g, 0.05 mole) and the diethyl ester (12 g, 0.06 mole) [from the condensation of Norcamphor with diethyl succinate followed by re-esterification, Example 1.1] in dry tetrahydrofuran (50 ($cm^3$) was added dropwise with stirring over 30 minutes to a solution of potassium t-butoxide (6.5 g, 0.06 mole) in dry tetrahydrofuran (250 $cm^3$), cooled in an ice-salt bath. When the addition was complete the reaction mixture was stirred (1 h), water (0.5 l) added, and tetrahydrofuran removed under reduced pressure on a rotatory evaporator.

The aqueous solution was acidified with concentrated hydrochloric acid and extracted with ether (2 x 150 $cm^3$). The ether extract was dried ($MgSO_4$), filtered, and the solvent evaporated. 10% Ethanolic potassium hydroxide (75 $cm^3$) was added to the residue, and the mixture was boiled (1 h) and left overnight at room temperature. The potassium salt of the diacid was filtered off, dissolved in water (300 $cm^3$) and acidified with concentrated hydrochloric acid. The liberated acid was extracted into ether (2 x 35 $cm^3$), dried ($MgSO_4$) and filtered. Acetyl chloride (50 $cm^3$) was added to the ethereal filtrate. After 2 hours, solvent was removed. The residual oil partly crystallised. The crystals were washed with isopropanol to give one of the (E) isomers of the desired fulgide as pale yellow crystals (0.4 g) with about 10% of other isomers. A further crop of crystals has been obtained from the isopropanol.

Example 12

**Production of Photoactive Fulgides (Formulae IIIa and IIIb; F>C* = bicyclo[2.2.1]heptan-6-ylidine; $P^1$ = 2-methyl-5-p-methoxyphenyl-furan-3-yl; $P^2$ = methyl; A = O)**

A solution of 2-methyl-3-acetyl-5-(methoxyphenyl)-furan (25 g, 0.11 mole) and 28.7 g, 0.11 mole of the condensation of diethyl succinate and Norcamphor in tetrahydrofuran (100 $cm^3$) was added dropwise (5 h) with stirring to potassium t-butoxide (14.6 g, 0.13 mole) in dry tetrahydrofuran (500 $cm^3$). The reaction mixture was stirred at room temperature overnight. Water (100 $cm^3$) was added and the solvent removed. The aqueous solution was acidified and extracted with ether. The ether layer was dried ($MgSO_4$) and filtered. The filtrate, on slow evaporation, gave solid half ester (13.2 g) and from the ether solution a deep red impure oily half ester (33.3 g) was obtained. The solid half ester (13.2 g) was hydrolysed by boiling with 10% ethanolic potassium hydroxide (300 $cm^3$) for 4 hours. On cooling and standing, the dipotassium salt separated. It was dissolved in water and acidified with concentrated hydrochloric acid. The resulting diacid (8.12 g) is extremely sensitive to ultraviolet light and colours to purple in unfiltered sunlight.

The diacid (8.12 g) was suspended in chloroform (100 $cm^3$) and stirred (12 h) with acetyl chloride (35 $cm^3$). Solvent was removed and the residue was triturated with isopropanol (20 $cm^3$). The solid was filtered off and is being recrystallised from a 1:1 mixture of ethyl acetate and petroleum (b.p. 80-100 °C). The oily half ester (33.3 g) was boiled (4 h) with 10% ethanolic potassium hydroxide (500 $cm^3$). Work up, as before, gave the diacid (5 g) which was dissolved in chloroform (20 $cm^3$) and treated with acetyl chloride (25 $cm^3$) and stirred for 12 hours.

Solvent was removed and the residue was dissolved in petroleum (b.p. 80-100 °C). On standing with slow evaporation, crystals are separating from the highly coloured solution.

Example 13

**Preparation of Intermediate - 2-5-Dimethyl-(2-Methylpropanoyl)furan**

2-Methylpropanoic anhydride (79.1 g, 0.5 mole) was added to a stirred solution of 2,5-dimethylfuran (42.3 g, 0.44 mole) in dichloromethane (400 $cm^3$) cooled to 0 °C. Tin (IV) chloride (14.6 g, 0.44 mole) in dichloromethane (50 $cm^3$) was added dropwise with stirring over 4 hours and the temperature of the reaction mixture was maintained at 0 °C. The deep red solution was stirred for a further 30 minutes and then poured into a mixture of crushed ice (150 g) and concentrated hydrochloric acid (100 $cm^3$). The organic layer was separated, washed with water, dilute sodium carbonate and water again, dried ($MgSO_4$) and filtered. Solvent was removed and the residual liquid was fractionally distilled, b.p. 180 °C at 1 mm. 2,5-Dimethyl(2-methylpropanoyl)furan was obtained as a pale yellow crystalline solid (58.9 g 81%).

A repeat preparation on a similar scale gave the 2,5-dimethyl-(2-methylpropanoyl)furan in 84% yield after fractional distillation.

Example 14

**Preparation of Photoactive Fulgides of Formulae IIIa and IIIb ( $\overline{F}$>C* = 4,7,7-trimethyl-bicyclo[2.2.1]-heptan-1-ylidine [derived from isofenchone], $P^1$ = 2,5-dimethylfuran-3-yl; $P^2$ = methyl; A = O)**

14.1 Preparation of isofenchone

α-Fenchyl alcohol (b.p. 201-2 °C) (295 g, 1.95 mole) was mixed with potassium hydrogen sulphate (50 g) and distilled slowly (3 h) so that the product distilled below 180 °C. The distillate, which consisted mainly of α-, β-, and -fenchenes (b.p. 157-9, 150-154, and 145-7 °C respectively), was added to a solution of acetic acid (550 $cm^3$), concentrated sulphuric acid (50 $cm^3$), and water (40 $cm^3$). The mixture was boiled (30 minutes), cooled, and diluted with water (3 l). The product was extracted into ether (3 x 400 $cm^3$) and the combined ether extracts were washed with water and then 1M sodium carbonate solution. The ether extracts were dried ($MgSO_4$), filtered and solvent removed, leaving an oil (270 g) which was mainly isofenchyl acetate.

The isofenchylacetate was dissolved in ethanol (800 $cm^3$) and aqueous potassium hydroxide [prepared from potassium hydroxide (120 g) and water (200 $cm^3$) added and boiled (15 h). Ethanol was removed under reduced pressure and water (1 l) was added). Isofenchyl alcohol was extracted with ether (3 x 0.5 l) and the combined ether extracts were dried ($MgSO_4$) and filtered. Solvent was removed leaving isofenchyl alcohol (225 g). The isofenchyl alcohol (180 g) was dissolved in ether (0.751 l) and added to crushed ice (1.5 k) in a 5 l flask, cooled in an ice/salt bath. Chromic acid [from sodium chromate (240 g) in water (1.2 l) and concentrated sulphuric acid (300 g)] was added rapidly over 30 minutes with vigorous stirring.

The ether layer was separated and the aqueous layer was extracted with ether (2 x 250 $cm^3$). The ether layers were combined and washed with water and then dilute sodium carbonate solution. The ether layer was dried ($MgSO_4$), filtered and solvent removed. The residual oil was fractionally distilled and isofenchone obtained as a colourless oil (86 g) b.p. 197-205 °C.

14.2 Condensation of isofenchone with dimethyl succinate

Isofenchone (76 g, 0.5 mole) and dimethylsuccinate (101 g, 0.7 mole) dissolved in tetrahydrofuran (100 $cm^3$) was added with rapid stirring to a solution of potassium t-butoxide (75 g, 0.7 mole) in tetrahydrofuran (250 $cm^3$). When the addition was complete, the reaction mixture was stirred (3 h) tetrahydrofuran removed under reduced pressure, and water (0.75 l) added. The mixture was extracted with ether (500 $cm^3$). The ether layer was discarded and the aqueous layer was acidified and extracted with ether (2 x 500 $cm^3$). The solid self-condensation product of dimethyl succinate separated out and was filtered off. The combined ether extracts were dried ($MgSO_4$), filtered and the solvent removed. The crude half ester (52 g) was dissolved in methanol (100 $cm^3$) and heated to boiling. Thionyl chloride (25 g) was added dropwise to this boiling solution over 3 hours. The reaction mixture was boiled for a further 2 hours and the solvent removed. The residual oil was dissolved in ether (200 $cm^3$), extracted with dilute sodium hydroxide, washed with water, dried ($MgSO_4$) and filtered. Solvent was removed and the residual oil was fractionally distilled. The fraction, b.p. 130-155 °C at 0.2 mm, (23 g) contained the required dimethyl ester.

14.3 Preparation of Fulgides

A solution of the crude diester (23 g) and 3-acetyl-2,5-dimethyl-furan (16.5 g, 0.12 mole) and added dropwise to a suspension of potassium t-butoxide (14.3 g, 0.13 mole) in toluene (125 $cm^3$) cooled in an ice/bath salt. The mixture was stirred (2 h) and water (500 $cm^3$) added. The aqueous layer was separated, acidified with concentrated hydrochloric acid and extracted with ether (2 x 250 $cm^3$). The combined ether extracts were dried ($MgSO_4$), filtered and solvent removed and the residual half ester hydrolysed by boiling (2 h) with 10% ethanolic potassium hydroxide (150 $cm^3$). No potassium salt separated after standing for 2 days. Ethanol was removed, water (200 $cm^3$) added and the solution acidified with concentrated hydrochloric acid the liberated acids were extracted into ether (2 x 200 $cm^3$), dried ($MgSO_4$), filtered and the solvent removed. The residual oil was chromatographed on a column of silica gel using petroleum (b.p. 60-80 °C) as eluant. Solvent was removed and the remaining pale yellow oil (6.2 g) was treated with acetyl chloride (25 $cm^3$), left for 30 minutes and solvent removed. The residual fulgide was subjected to flash chromatography on silica gel using petroleum (b.p. 60-80 °C) as eluant for the first fraction and 1:20 mixture of ethyl acetate:petroleum for the second fraction. The first fraction gave a photochromic isomer of the title fulgides, pale yellow needles (90 mg) shown to have an (E) configuration of the furyl group from n.m.r. studies, Its red photocoloured form has a low quantum efficiency for bleaching.

The second fraction is also photochromic and its photocoloured form has a much higher quantum efficiency for bleaching, indicating that the configuration of the isofenchylidene group influences the bleaching efficiencies.

Example 15

**Production of Photoactive Fulgides (Formulae IIIa and IIIb; F>C* = bicyclo[2.2.1]heptan-6-ylidine; $P^1$ = 2-methyl 5-(thiophen-2-yl)-furan-3-yl; $P^2$ = methyl; A = O)**

The procedure of Example 1.3 was repeated, but using 2-methyl-3-acetyl-5-(thiophen-2-yl)-furan (20.6 g) in place of 2,5-dimethyl-3-acetyl furan.

The resulting photoactive compounds of formulae (IIIa) and (IIIb) coloured to purple on exposure to U.V.

Example 16

**Production of Photoactive Fulgides (Formulae IIIa and IIIb; F>C* = bicyclo[2.2.1]heptan-6-ylidine; $P^1$ = 2-methyl 5-(thiophen-3-yl)-furan-3-yl; $P^2$ = methyl; A = O)**

The procedure of Example 1.3 was repeated, but using 2-methyl-3-acetyl-5-(thiophen-3-yl)-furan (20.6 g) in place of 2,5-dimethyl-3-acetyl furan.

The resulting photoactive compounds of formulae (IIIa) and (IIIb) coloured to purple on exposure to U.V.

Example 17

**Production of Photoactive Fulgides (Formulae IIIa and IIIb; F>C* = bicyclo[2.2.1]heptan-6-ylidine; $P^1$ = 2-methyl-5-(3-methylthiophen-2-yl)-furan-3-yl; $P^2$ = methyl; A = O)**

The procedure of Example 1.3 was repeated, but using 2-methyl-3-acetyl-5-(3-methylhiophen-2-yl)-furan (22 g) in place of 2,5-dimethyl-3-acetyl furan. The resulting photoactive compounds of formulae (IIIa) and (IIIb) coloured to purple on exposure to U.V.

Example 18

**Production of Photoactive Fulgides (Formulae IIIa and IIIb; F>C* = bicyclo[2.2.1]heptan-6-ylidine; $P^1$ = 2-methyl-5-(2,5-dimethyl-thiophen-2-yl)-furan-3-yl; $P^2$ = methyl; A = O)**

The procedure of Example 1.3 was repeated, but using 2-methyl-3-acetyl-5-(thiophen-2-yl)-furan (20.6 g) in place of 2,5-dimethyl-3-acetyl furan.

The resulting photoactive compounds of formulae (IIIa) and (IIIb) coloured to purple on exposure to U.V.

Example 19-22

The following compounds were produced by following the procedures of Examples 15-18, but using tricyclodeca[5.2.1.$O^{2,6}$]-8-one in place of norcamphor.

Formulae IIIa and IIIb; F>C* = tricyclodeca[5.2.1.$O^{2,6}$]-8-ylidine; $P^1$ = 2-methyl 5-(thiophen-2-yl)furan-3-yl; $P^2$ = methyl; A = O

Formulae IIIa and IIIb; F>C* = tricyclodeca[5.2.1.$O^{2,6}$]-8-ylidine; $P^1$ = 2-methyl 5-(thiophen-3-yl)-furan-3-yl; $P^2$ = methyl; A = O

Formulae IIIa and IIIb; F>C* = tricyclodeca[5.2.1.$O^{2,6}$]-8-ylidine; $P^1$ = 2-methyl-5-(3-methyl-thiophen-2-yl)-furan-3-yl; $P^2$ = methyl; A = O

Formulae IIIa and IIIb; F>C* = tricyclodeca[5.2.1.$O^{2,6}$]-8-ylidine; $P^1$ = 2-methyl-5(2,5-dimethyl-thiophen-2-yl)-furan-3-yl; $P^2$ = methyl; A = O

The following structural formula represents the cyclised product of photoactive change which may be formed from compounds of formula IIIa and IIIb

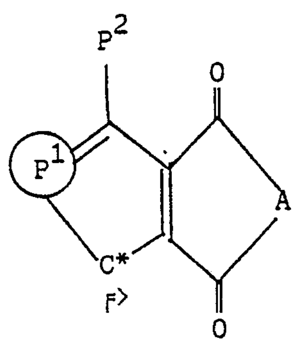

or

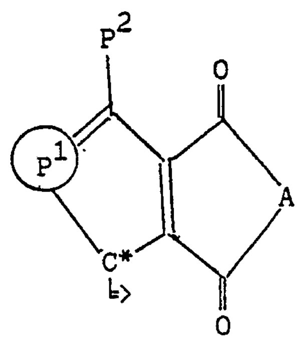

wherein $P^2$, A, F>C*, and F>C* are as defined above and (P1) is the residue of $P^1$ (as defined above). For example, where $P^1$ is unsubstituted or substituted 3-furyl, 3-thienyl or 3-pyrryl, (P1) represents

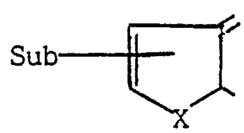

wherein X is oxygen, nitrogen or sulphur and S represents the optional substitution. Where $P^1$ is unsubstituted or substituted 3-benzofuryl, or 3-benzothienyl, (P1) represents

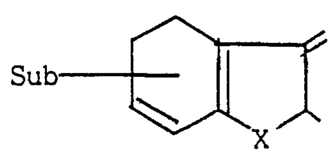

wherein X is oxygen or sulphur and Sub represents the opional substitution. An example of a specific cyclised compound is

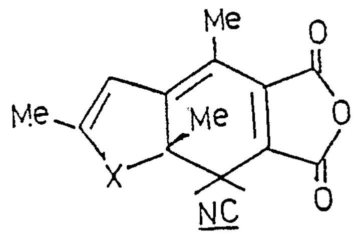

(NC = residue of norcamphor, i.e. bicyclo[2.2.1]heptan-6-ylidine)

## Claims

1. A photoactive compound having the general formula

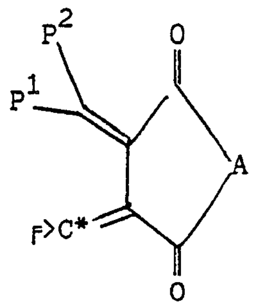

or

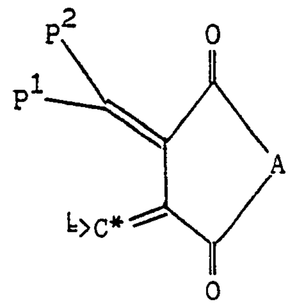

(IIIa) (IIIb)

in which
A represents oxygen or $NR^1$ wherein $R^1$ represents hydrogen, alkyl having 1 to 20 carbon atoms, cycloalkyl having 5 to 12 carbon atoms, aralkyl having 7 to 9 carbon atoms, aryl having 6 to 14 carbon atoms, which may be substituted with one or more halogen or alkoxy groups having 1 to 20 carbon atoms, or alkaryl having 7 to 22 carbon atoms;
$P^1$ represents a 3-furyl, a 3-thienyl, a 3-pyrryl, a 3-benzofuryl or a 3-benzothienyl group, said 3-furyl, 3-thienyl and 3-pyrryl groups being unsubstituted or substituted in the 2-position and/or the 5-position, and said 3-benzofuryl and 3-benzothienyl groups being unsubstituted or substituted in the 2-position, said optional 2-substituents being selected from alkyl groups having 1 to 20 carbon atoms and aralkyl groups having 7 to 12 carbon atoms, and said optional 5-substituents being selected from (i) alkyl groups having 1 to 20 carbon atoms, (ii) cycloalkyl groups having 3 to 12 carbon atoms, (iii) cycloalkenyl groups having 3 to 12 carbon atoms, (iv) aryl groups having 6 to 14 carbon atoms, which aryl groups may be unsubstituted or substituted with (a) one or more alkoxy groups (b) with a group of the formula $-NR_7R_8$ wherein $R_7$ and $R_8$ each represent hydrogen or alkyl having 1 to 20 carbon atoms or together with the nitrogen atom to which they are attached represent a 1-pyrrolidine, a 1-piperidine, or a morpholine group, or (c) with one or more halogen atoms, (v) aralkyl groups having 7 to 12 carbon atoms, (vi) halogen atoms, and (vii) heterocyclic groups selected from 2-thienyl, 3-thienyl, 2-furyl and 3-furyl groups, said last-mentioned 2-thienyl, 3-thienyl, 2-furyl and 3-furyl groups being unsubstituted or substituted by one or more $C_{1-3}$ alkyl groups or halogen atoms;
$P^2$ represents alkyl having 1 to 20 carbon atoms, cycloalkyl having 3 to 12 carbon atoms, aralkyl having 7 to 9 carbon atoms, aryl having 6 to 14 carbon atoms which may be unsubstituted or substituted with one or more halogen atoms, or alkaryl having 7 to 22 carbon atoms; and
F>C* and L>C* represent a substituted or unsubstituted bridged polycyclic hydrocarbon residue containing from 7 to 20 carbon atoms in a polycyclic system, said residue having a plane of asymmetry which is parallel to the plane which includes the single bonds extending from carbon atom C* and the

anhydride or imide ring, any substituents on the bridged polycyclic hydrocarbyl residue being selected from alkyl groups having 1 to 4 carbon atoms, halogen atoms and hydroxy groups.

2. A compound according to Claim 1 wherein the moieties F>C* and E>C* have the structure

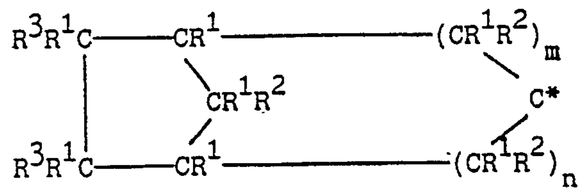

(IV)

wherein each $R^1$ and $R^2$ is independently selected from hydrogen and $C_{1-4}$ alkyl, each $R^3$ is independently selected from hydrogen and $C_{1-4}$ alkyl or the two $R^3$ groups together form an alkylene group having up to 6 carbon atoms and one of m and n is 1 and the other is 1 or 0.

3. A compound according to Claim 2 wherein one of m and n is 1 and the other is 0.

4. A compound according to Claim 3 wherein the symbols F>C* and E>C* represent the structure

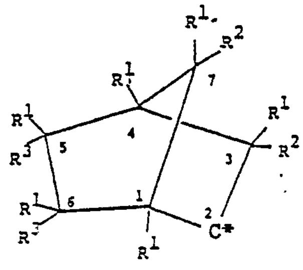

wherein $R^1$, $R^2$ and $R^3$ are as defined in Claim 2.

5. A compound according to any of Claims 2 to 4 wherein each $R^1$, $R^2$ and $R^3$ are hydrogen or methyl.

6. A compound according to Claim 4 wherein one, two or three of the carbon atoms at positions 1, 4, 5 and 6 are substituted by a methyl group and the remaining substituents $R^1$, $R^2$ and $R^3$ are all hydrogen.

7. A compound according to Claim 1 wherein the moieties F>C* and E>C* are is selected from groups of the following formulae:

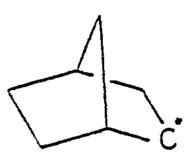

Va

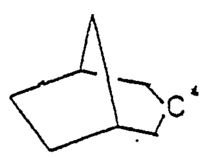

Vb

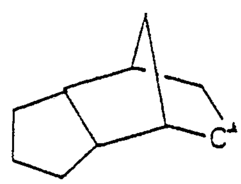

Vc

and

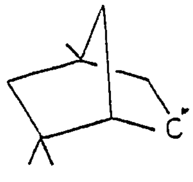

Vd

8. A compound according to any preceding claim wherein $P^1$ represents a 2,5-dimethylfuran-3-yl group, a 2,5-dimethyl thiophen-3-yl group, a 2-methyl-5-phenyl-thiophen-3-yl group, a 2-methyl-5-(p-methoxyphenyl)-furan-3-yl group, a 2-methyl-5-(p-fluorophenyl)-furan-3-yl group, a 2-methyl-5-(thiophen-3-yl)-furan-3-yl group, or a 2-methyl-5-(thiophen-2-yl)-furan-3-yl group.

9. A compound according to any preceding claim wherein $P^2$ represents a methyl group.

10. A compound according to any preceding claim wherein F >C* and L >C* represent a bicyclo-[2.2.1]hept-2-ylidene group, a bicyclo[3.2.1]octan-3-yl group, a tricyclodeca[5.2.1.O$^{2,6}$]-8-yl group or a 4,7,7-trimethylbicyclo[2.2.1]heptan-2-yl group.

11. A compound having the formula

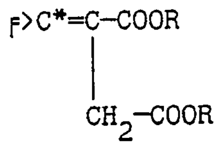

(IXa)

or

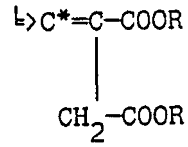

(IXb)

wherein R and R′ which may be the same or different represent hydrogen atoms or alkyl groups having 1 to 4 carbon atoms and F >C* and L>C* are as defined in Claim 1.

12. A compound according to Claim 4 where and F>C* and L>C* are as defined in any of Claims 2 to 8.

13. A process for producing a condensation product between a lower $C_{1-4}$ alkyl ester of succinic acid (or a derivative of succinic acid in which at least one of the hydrogen atoms of one of the methylene groups is replaced by a substituent which does not interfere with the reaction) and an aldehyde or ketone which comprises reacting said ester and said aldehyde or ketone in the presence of an aromatic hydrocarbon and an alkali metal alkoxide.

14. A process according to Claim 13 wherein the aromatic hydrocarbon is toluene.

15. A process according to Claim 13 or Claim 14 wherein the alkali metal alkoxide is an alkali metal t-butoxide.

16. A process according to Claim 15 wherein the alkali metal alkoxide is potassium t-butoxide.

17. A process for producing a compound of formula

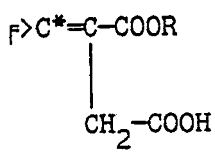

(VIII)

wherein R is $C_{1-4}$ lower alkyl and F>C* is as defined in ay of Claims 1 to 8 which comprises reacting a di-lower alkyl succinate of general formula

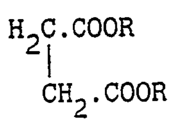

(VII)

wherein R is as defined above with a ketone of formula

F>C* = CO (XIII)

wherein F>C* is as defined above, characterised in that the reaction is carried out in a reaction medium comprising toluene and in the presence of potassium t-butoxide as catalyst.

18. A process according to Claim 17 wherein the resulting compound of formula (VIII) is then esterified to form a compound of formula (IX)

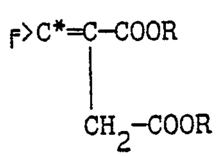

(IX)

and reacted with a ketone of formula $P^1COP^2$, wherein $P^1$ and $P^2$ are as defined in any of Claims 1 to 9 to form a compound of formula (X)

19. A process for producing a compound of Formula (IIIa) or (IIIb) as defined in any of Claims 1 to 10 which comprises one of the following reaction sequences

Sequence 1

(i) reacting ketone (VI) with a di-lower alkyl succinate (VII, R = lower $C_{1-4}$ alkyl)

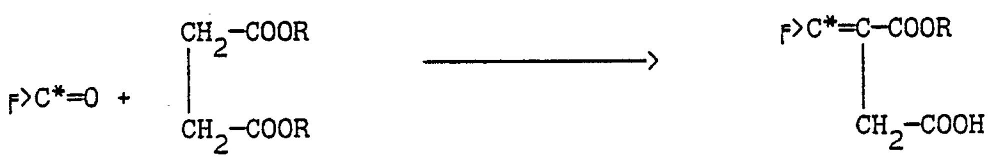

(VI) (VII) (VIII)

to yield condensation product VIII,

(ii) esterifying condensation product VIII to form the diester (IX, R = lower alkyl) and reacting the diester with a ketone $P^1COP^2$

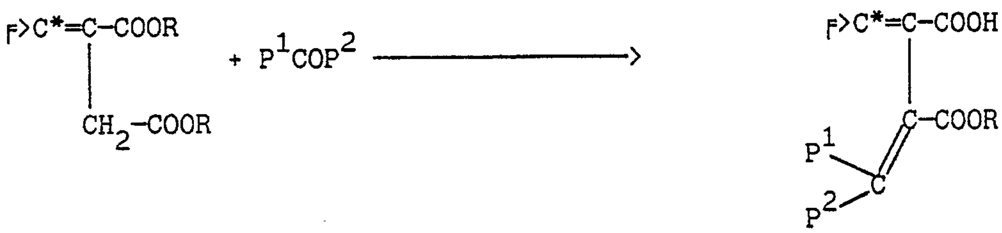

(IX) (X)

(iii) hydrolysing half-ester X to form the corresponding dicarboxylic acid, and

(iv) converting the dicarboxylic acid to the desired compounds of formulae IIIa and IIIb by treatment with a dehydrating agent to form a compound in which A is oxygen, and optionally converting said compound(s) into a compound in which A represents $NR^1$ by reacting with an amine $H_2NR^1$;

Sequence 2

(i) Reacting a ketone $P^1COP^2$ with a di-lower alkyl succinate (VII, R = lower $C_{1-4}$ alkyl)

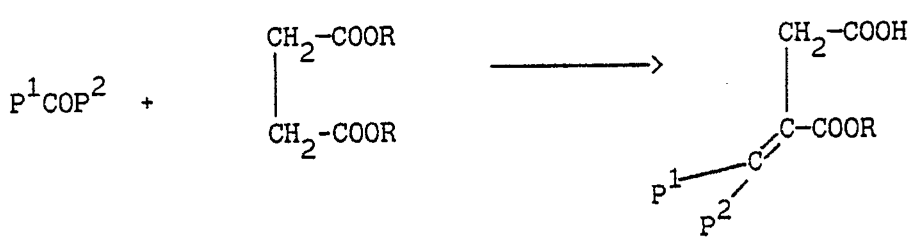

(VII) (XI)

to yield a condensation product XI.

(ii) esterifying condensation product XI to form the diester (XII, R = lower alkyl) and reacting the diester with a ketone (VI)

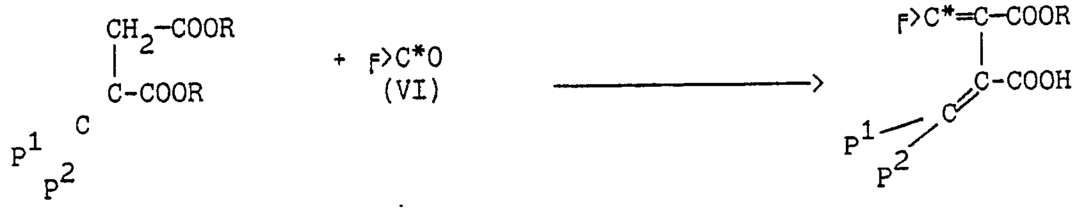

(VI)

(XII)

(XIII)

(iii) hydrolysing half-ester (XIII) to form the corresponding dicarboxylic acid, and

(iv) converting the dicarboxylic acid to the desired compounds of formulae IIIa and IIIb by treatment with a dehydrating agent to form a compound in which A is oxygen, and optionally converting said compound(s) into a compound in which A represents $NR^1$ by reacting with an amine $H_2NR^1$;

20. A process for producing a photoactive compound of Formula (IIIa) or (IIIb) as claimed in any of Claims 1 to 10 from a non-photoactive geometric isomer of (IIIc) or (IIId)

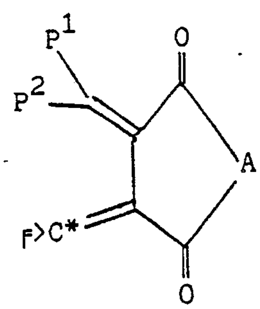

(IIIc)

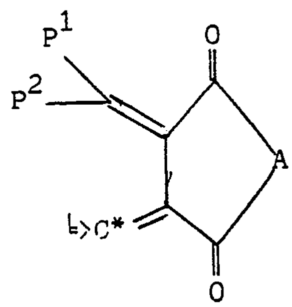

(IIId)

which comprises exposing said non-photoactive geometric isomer to ultraviolet light.

21. A process according to Claim 20 wherein said ultraviolet light has a wavelength in the range 200 to 400 nm.

22. A process according to Claim 20 or Claim 21 wherein compounds of Formula (IIIa) or (IIIb) formed as a result of said exposure undergo a photoactive change, and the product(s) of said photoactive change are re-converted to compounds of formula (IIIa) or (IIIb) by exposing them to white light or monochromatic light in the visible range.

23. Use of a compound as claimed in any of Claims 1 to 10 in the manufacture of image and data recording media.

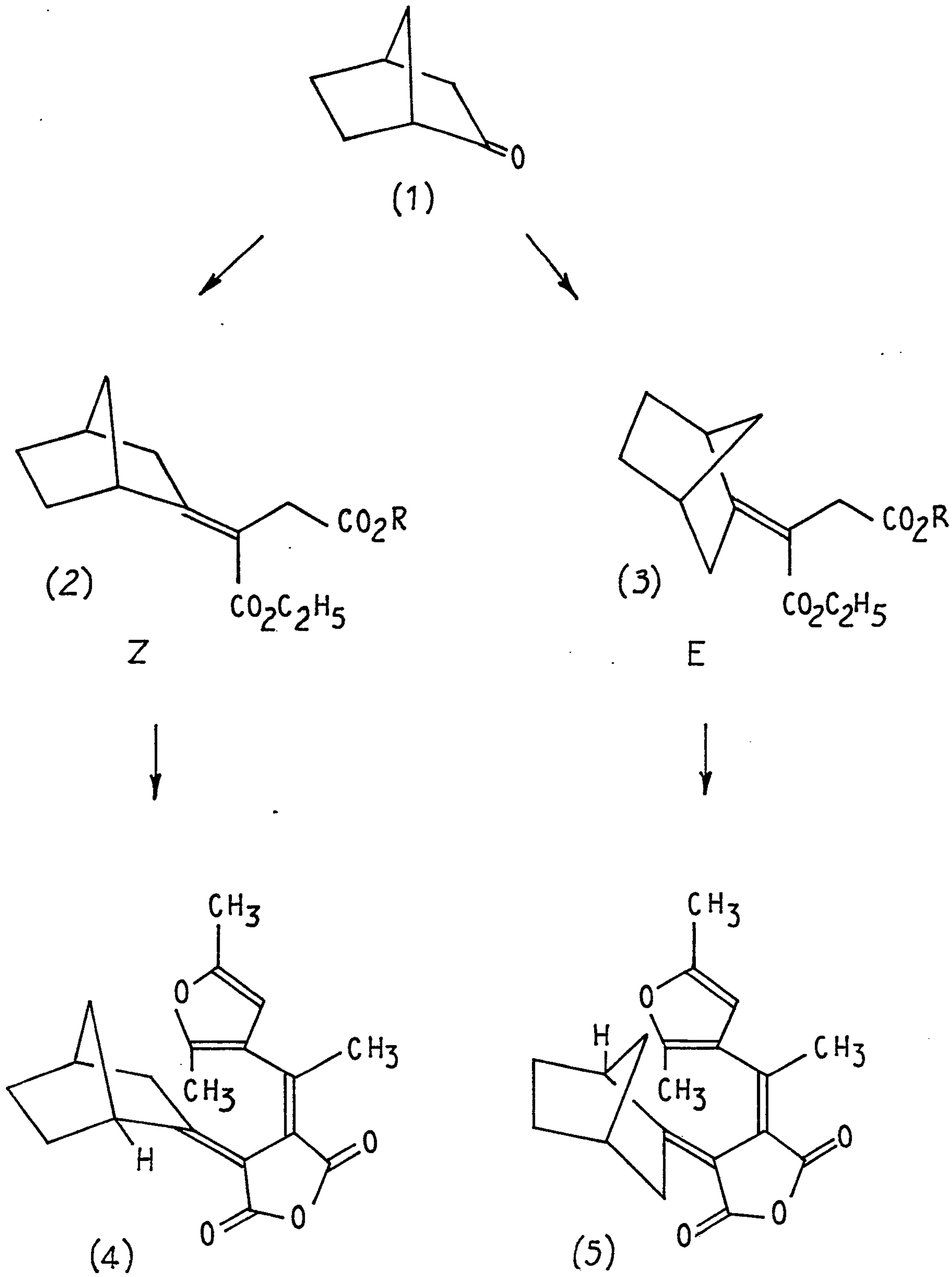

(1)

(2) Z

(3) E

(4) E,Z

(5) E,E

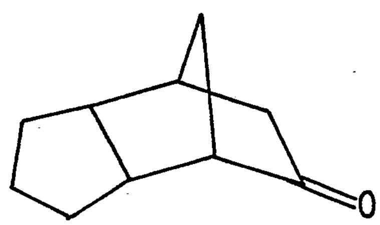

(6)

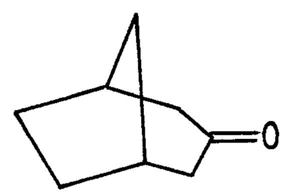

(7)

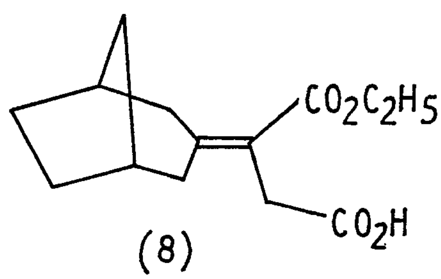

(8)

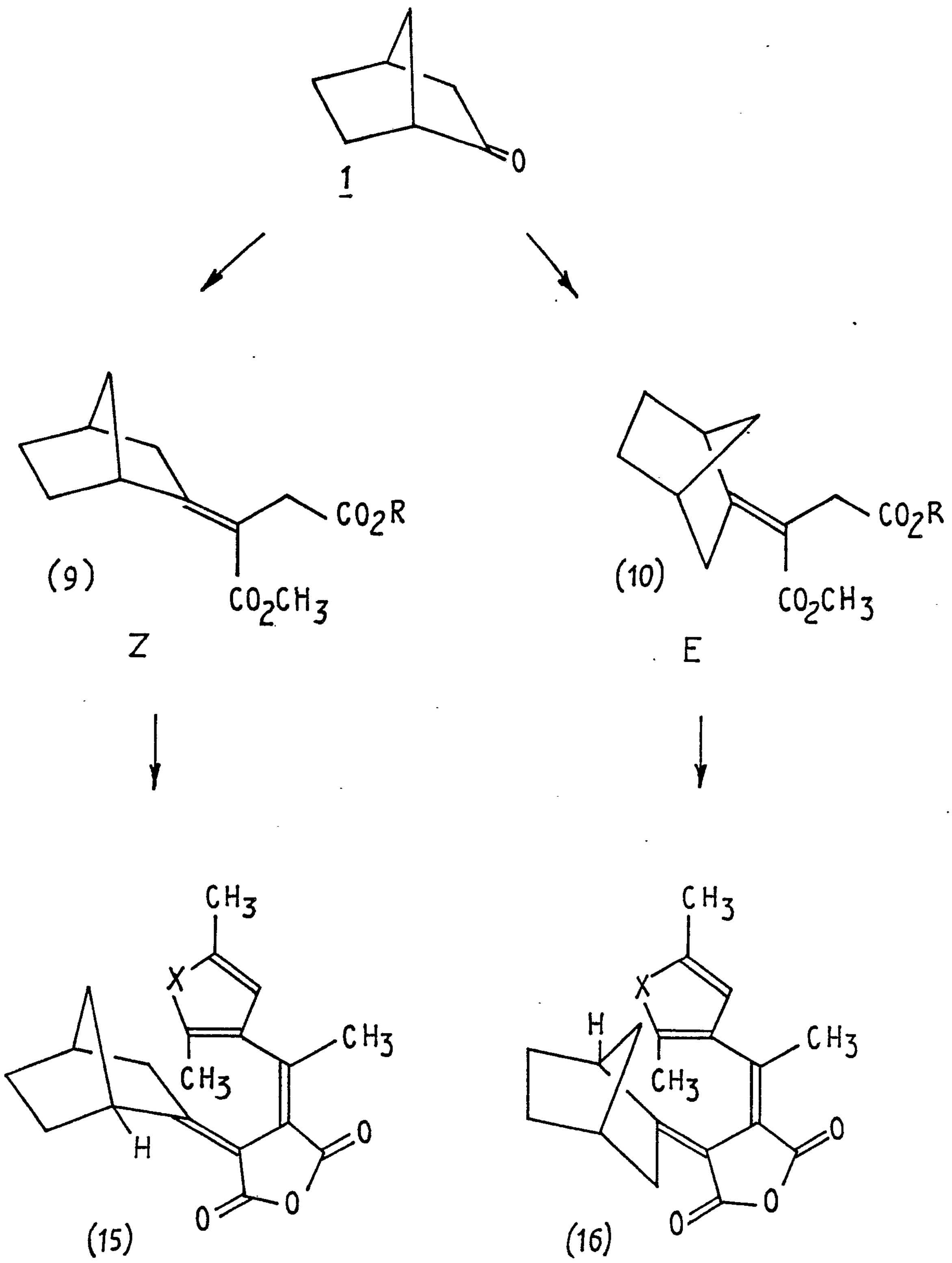

1

(9) Z

(10) E

(15) E,Z

(16) E,E

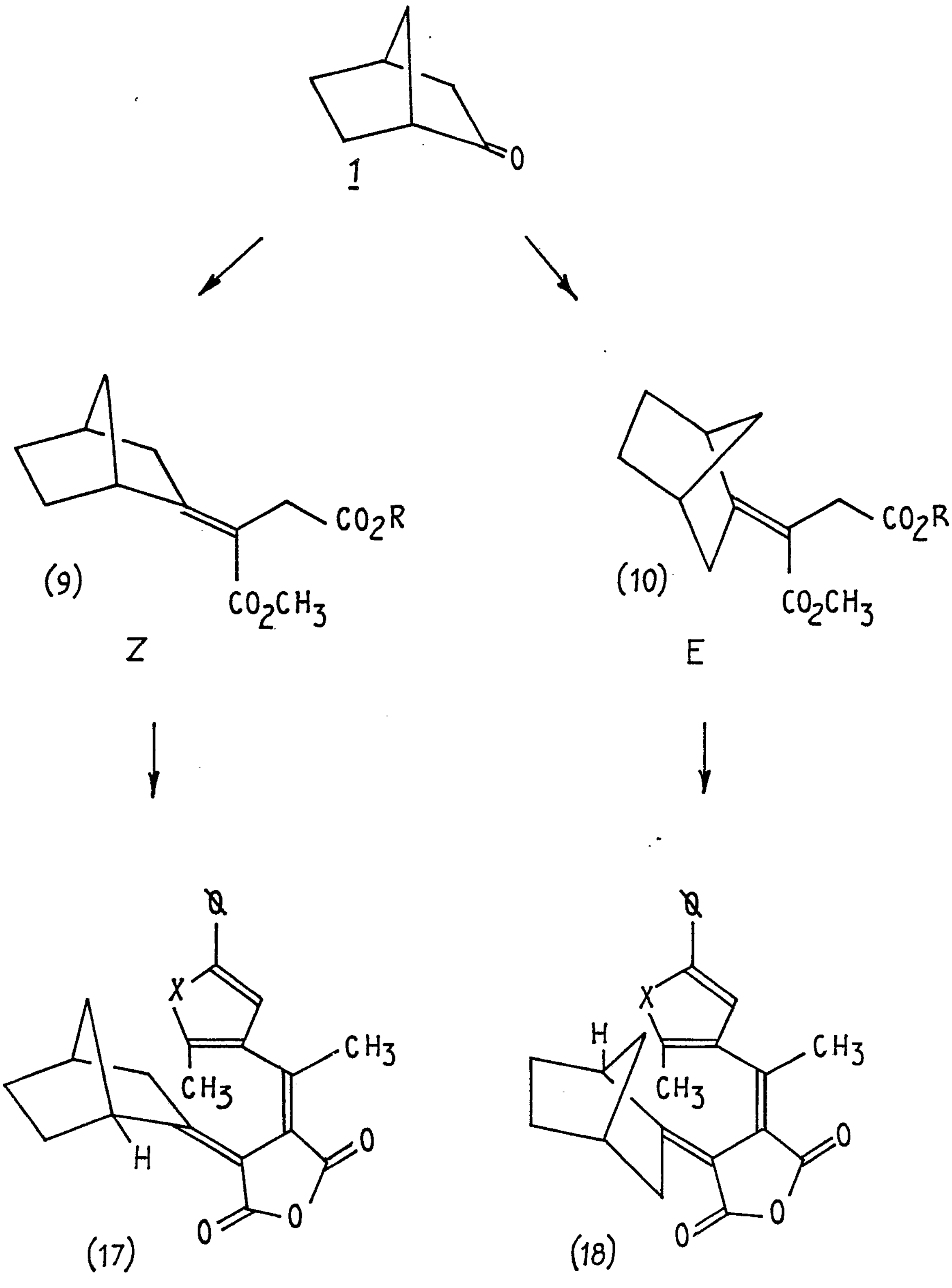

1

(9) Z

(10) E

(17) E,Z

(18) E,E

[Ø = phenyl]

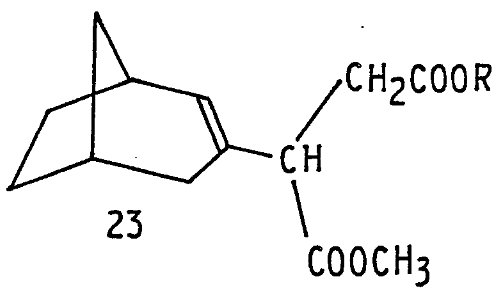

23

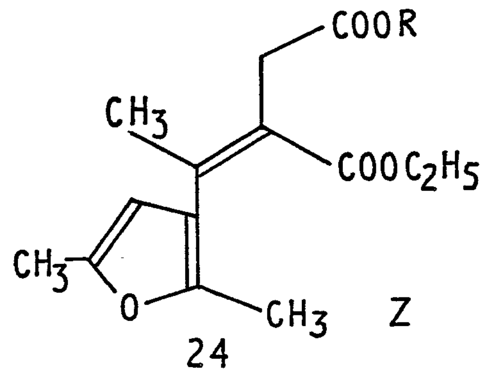

24 Z

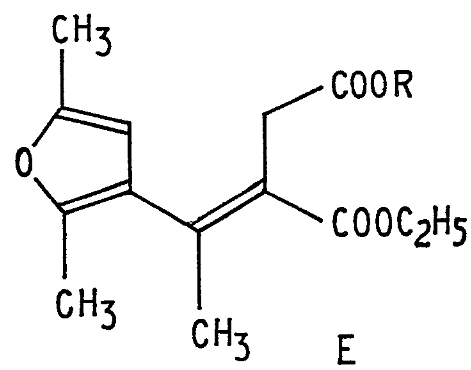

E

25

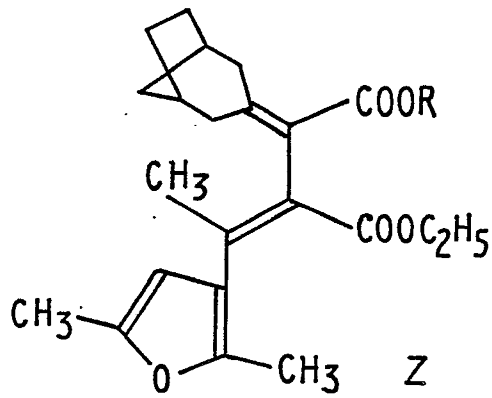

Z

(26)

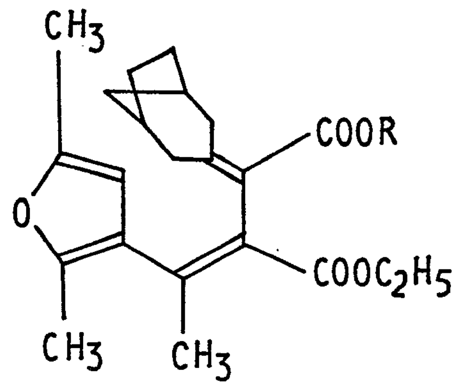

E

(27)

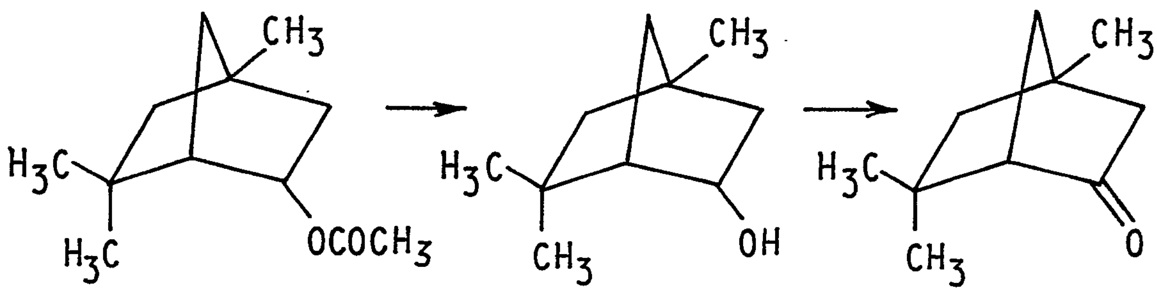

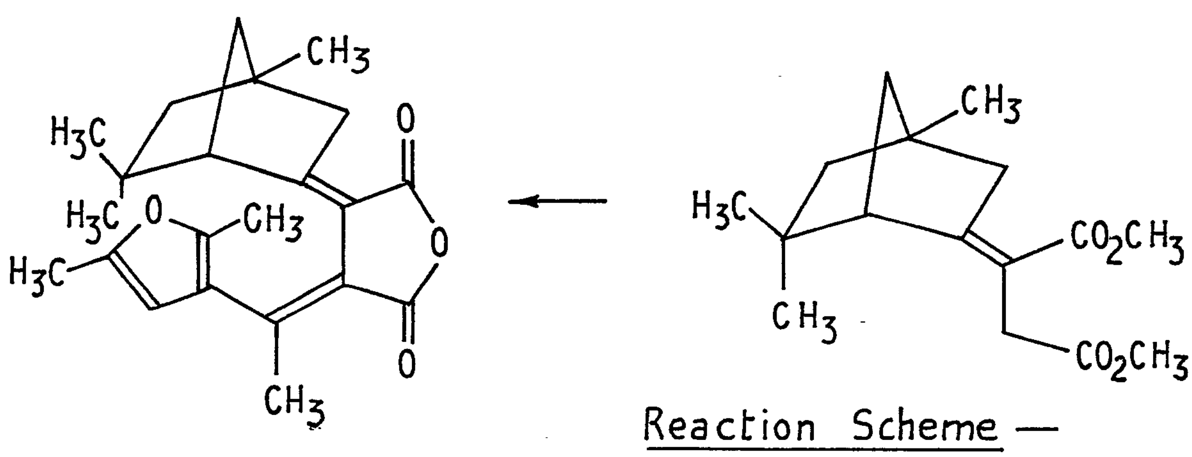

Reaction Scheme —
Example 14